# EUROPÄISCHE PATENTSCHRIFT

(11) **EP 2 785 309 B1**
(45) Veröffentlichungstag und Bekanntmachung des Hinweises auf die Patenterteilung: **12.07.2017**
(21) Anmeldenummer: 12790869.7
(22) Anmeldetag: 20.11.2012
(51) Int. Cl.: A61K 8/04, A61K 8/42, A61Q 15/00, A61K 8/06

(54) **KOSMETISCHES AEROSOLSPRAY MIT ANHALTENDEM FRISCHEEFFEKT**
COSMETIC AEROSOL SPRAY WITH LASTING FRESHNESS EFFECT
SPRAY AÉROSOL COSMÉTIQUE À EFFET RAFRAÎCHISSANT DURABLE

(30) Priorität: 02.12.2011 DE 102011087662
(43) Veröffentlichungstag der Anmeldung: 08.10.2014
(73) Patentinhaber: Henkel AG & Co. KGaA, 40589 Düsseldorf (DE)
(72) Erfinder: BANOWSKI, Bernhard, 40597 Düsseldorf (DE); BREUER, Imme, 40474 Düsseldorf (DE)
(86) Internationale Anmeldenummer: PCT/EP2012/073031
(87) Internationale Veröffentlichungsnummer: WO 2013/079349

(56) Entgegenhaltungen:
- WO-A2-2010/149798

## Beschreibung

Die vorliegende Anmeldung beschreibt als Aerosol versprühbare kosmetische Wasser-in-Öl-Emulsionen, die einen anhaltenden Frischeeffekt aufweisen.

Ein Aerosol ist ein disperses System, bei dem ein Feststoff oder eine Flüssigkeit in einem Gas sehr fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Treibmittel-Zubereitungsgemisch durch eine feine Düse, das Treibmittel verdunstet und hinterlässt das fein verteilte Sprühgut als Aerosol.

Ein technisch wichtiges Gebiet für die Anwendung kosmetischer Aerosolsprays ist der Bereich der deodorierenden kosmetischen Erzeugnisse. Gängige Deodorantsprayzusammensetzungen liegen dabei häufig als wasserfreie ethanolische Lösungen des deodorierenden Wirkstoffes vor. Ein Nachteil dieser ethanolischen Lösungen ist bei entsprechend prädisponierten Personen mit empfindlicher Haut eine hautreizende Wirkung. Auch auf durch eine Rasur mechanisch gereizter Haut führt die Applikation der gängigen ethanolischen Lösungen zu einem sehr unangenehmen Brennen. Ein weiterer Nachteil ethanolischer Lösungen ist, dass die Einarbeitung wasserhaltiger oder wasserlöslicher Wirkstoffe, insbesondere von Antitranspirant- bzw. Deodorant-Wirkstoffen oder haarwuchshemmenden Wirkstoffen, die nicht auch in Ethanol löslich sind, nicht oder nur sehr eingeschränkt möglich ist.

Als alternative Angebotsform zu den ethanolischen Lösungen werden im Stand der Technik wasserfreie Suspensionen des pulverförmigen schweißreduzierenden Wirkstoffes, meist eines Aluminiumsalzes, neben dem Treibgas in einem flüssigen Träger, meist einem relativ flüchtigen Öl wie Cyclomethicone, diskutiert. Vor dem Versprühen muss die Suspension aufgeschüttelt werden. Ein Nachteil dieser Suspensionsaerosole ist das Risiko, dass die Ventil- bzw. Düsenbohrungen bei höheren Einsatzkonzentrationen des Salzes verstopfen. Es gab daher Versuche, das Antitranspirant-Salz in gelöster Form zu versprühen. Jedoch verursacht die Konfektionierung wässriger Antitranspirant-Salzlösungen in Treibmittel-haltigen Metalldosen große Korrosionsprobleme bei der Aerosolverpackung, so dass selbst bei lackierten Spraydosen unweigerlich Korrosionserscheinungen an der Dose auftreten.

Als weitere alternative Angebotsform beschreibt der Stand der Technik Wasser-in-Öl-Emulsionen (W/O-Emulsionen). So offenbart die deutsche Patentanmeldung DE 102006062499 A1 (Henkel) eine als Aerosolspray geeignete W/O-Emulsion.

Schweißhemmende W/O-Emulsionssprays weisen besonders hautpflegende Eigenschaften auf.

Beim Aufsprühen auf die Haut wird durch das Treibgas vorübergehend ein leicht kühlender Effekt erzeugt, der allerdings nicht anhält. Der kühlende Effekt, der durch die enthaltene wässrige Phase der Emulsion entsteht, ist bei einer W/O-Emulsion, bei der die wässrige Phase die innere Phase darstellt, kaum wahrnehmbar. Einen Haut kühlenden Effekt können neben leicht flüchtige Substanzen, die Verdunstungskälte erzeugen, auch durch solche Substanzen hervorgerufen werden, die die Thermorezeptoren in der Haut und den Schleimhäuten so stimulieren, dass ein kühler sensorischer Eindruck entsteht. Insbesondere der Rezeptor CMR-1 ("cold- and mentholsensitive receptor"), der zur Familie der TRP-Kanäle gehört, wird durch derartige Kühlwirkstoffe stimuliert, wodurch ein Kälteeindruck erzeugt wird. Der bekannteste derartige Kühlwirkstoff ist sicherlich L-Menthol. L-Menthol ist zwar ein recht effizienter Kühlwirkstoff, der aber diverse Nachteile aufweist: die kühlende Wirkung ist nicht sehr anhaltend, außerdem hat die Substanz einen deutlichen Eigengeruch, der eine variable Parfümierung der kosmetischen Produkte mit möglichst vielen verschiedenen Duftstoffen entgegensteht. Darüber hinaus kann L-Menthol bei empfindlichen Personen unerwünschte Hautreaktionen provozieren.

Aus WO 2010/149798A1 war eine Vielzahl kosmetischer Zusammensetzungen, enthaltend den Kühlwirkstoff 5-Methyl-2-(1-methylethyl)cyclohexyl-N-ethyloxamat, bekannt.

Der vorliegenden Anmeldung lag die Aufgabe zugrunde, eine für die Anwendung in einem Aerosolspray geeignete stabile Wasser-in-Öl-Emulsion mit einem Gehalt an schweißhemmenden Aluminiumsalzen bereitzustellen, die einen lang anhaltenden Kühl- und Frischeeffekt auf der Haut bewirkt und die vorstehend geschilderten Nachteile des Kühlwirkstoffs L-Menthol vermeidet. Unter "lang anhaltend" wird erfindungsgemäß ein Zeitraum von mindestens einer Stunde, bevorzugt von zwei bis mindestens vier Stunden nach Applikation des Produkts auf die Haut verstanden.

Es wurde festgestellt, dass die aufgeführte Aufgabenstellung durch schweißhemmende Wasser-in-Öl-Emulsions-Sprays, die 5-Methyl-2-(1-methylethyl)cyclohexyl-N-ethyloxamat als Kühlwirkstoff enthalten, gelöst wird.

Ein erster Gegenstand der vorliegenden Anmeldung ist daher ein kosmetisches Produkt, bestehend aus - einer Wasser-in-Öl-Emulsion, enthaltend
a) mindestens ein schweißhemmendes Aluminiumsalz,
b) mindestens einen Wasser-in-Öl-Emulgator mit einem HLB-Wert im Bereich von 1,5 - 6,5,
c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
d) mindestens ein kosmetisches Öl, das kein Riechstoff und kein ätherisches Öl ist,
e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat,
   - mindestens einem Treibmittel,
   - einer Aerosol-Abgabevorrichtung.

Sofern nicht anders angegeben, beziehen sich alle Mengenangaben in dieser Anmeldung auf das Gewicht der erfindungsgemäßen Treibmittel-freien Wasser-in-Öl-Emulsion.

Die erfindungsgemäßen Wasser-in-Öl-Emulsionen enthalten mindestens ein schweißhemmendes Aluminiumsalz.

Bevorzugte schweißhemmende Aluminiumsalze sind ausgewählt aus den wasserlöslichen adstringierenden anorganischen und organischen Salzen des Aluminiums bzw. beliebigen Mischungen dieser Salze. Alumosilicate und Zeolithe zählen erfindungsgemäß nicht zu den schweißhemmenden Aluminiumsalzen. Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind.

Besonders bevorzugte schweißhemmende Aluminiumsalze sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (= depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (= depolymerisierter) Form vorliegen kann.

Weiterhin bevorzugt sind Aluminiumsesquichlorhydrat, Aluminiumdichlorhydrat, oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG, Aluminiumsesquichlorohydrex-PEG oder Aluminium-PEG-dichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat.

Erfindungsgemäß besonders bevorzugte schweißhemmende Aluminiumsalze sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet. Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminiumsalze, enthaltend, bezogen auf ihr Gewicht, 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.

Besonders bevorzugte feste schweißhemmende aktivierte Salzzusammensetzungen enthalten, bezogen auf ihr Gewicht, 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 - 1:10, beträgt.

Weitere besonders bevorzugte feste schweißhemmende aktivierte Salzzusammensetzungen enthalten, bezogen auf ihr Gewicht, 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen enthalten, bezogen auf ihr Gewicht, 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Besonders bevorzugte erfindungsgemäße Wasser-in-Öl-Emulsionen enthalten den mindestens einen Antitranspirant-Wirkstoff in einer Gesamtmenge von 5 bis 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion.

In einer besonders bevorzugten Ausführungsform enthält die Wasser-in-Öl-Emulsion ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. In einer weiteren besonders bevorzugten Ausführungsform enthält die Wasser-in-Öl-Emulsion ein nicht-aktiviertes adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nichtaktivierte Aluminiumchlorohydrate sind beispielsweise Chlorhydrol^{®} (Summit-Reheis), ACH 303 (Summit-Reheis), Locron^{®} L (Clariant) oder Aloxicoll^{®} L (Giulini). Eine bevorzugte aktivierte Aluminiumchlorohydrat-Lösung ist z. B. Reach^{®} 501 von Summit-Reheis.

Ein bevorzugter Gegenstand der vorliegenden Anmeldung ist ein kosmetisches Produkt, bestehend aus - einer Wasser-in-Öl-Emulsion, enthaltend
a) mindestens ein schweißhemmendes Aluminiumsalz in einer Gesamtmenge von 5 - 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion,
b) mindestens einen Wasser-in-Öl-Emulgator mit einem HLB-Wert im Bereich von 1,5 bis 6,5,
c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
d) mindestens ein kosmetisches Öl, das kein Riechstoff und kein ätherisches Öl ist,
e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat
   - mindestens einem Treibmittel,
   - einer Aerosol Abgabevorrichtung.

Die erfindungsgemäßen Wasser-in-Öl-Emulsionen enthalten als zweiten wesentlichen Bestandteil mindestens einen Wasser-in-Öl-Emulgator. Als Wasser-in-Öl-Emulgator wird im Sinne der vorliegenden Anmeldung eine grenzflächenaktive Substanz angesehen, die unter Normalbedingungen einen HLB-Wert im Bereich von 1,5 bis 6,5, bevorzugt 4 bis 6,5, aufweist. "Normalbedingungen" sind im Sinne der vorliegenden Anmeldung eine Temperatur von 20 °C und ein Druck von 1013,25 hPa. Schmelzpunktangaben beziehen sich ebenfalls auf einen Druck von 1013,25 hPa. Wasser-in-Öl-Emulsionen, bei denen der mindestens eine Wasser-in-Öl-Emulgator b) in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion, enthalten ist, sind erfindungsgemäß bevorzugt.

Erfindungsgemäß bevorzugte Wasser-in-Öl-Emulgatoren sind Poly-(C₂-C₃)alkylenglycol-modifizierte Silicone, die mit C₄-C₁₈-Alkylgruppen hydrophob modifiziert sind, besonders bevorzugt Cetyl PEG/PPG-10/1 Dimethicone (frühere INCI-Bezeichnung Cetyl Dimethicone Copolyol), zum Beispiel erhältlich als Abil EM 90 oder in einer Mischung aus Polyglyceryl-4-isostearat, Cetyl PEG/PPG-10/1 Dimethicone und Hexyllaurat unter dem Handelsnamen Abil WE 09 von der Firma Evonik, sowie weiterhin bevorzugt Lauryl PEG/PPG-18/18 Methicone, zum Beispiel erhältlich als Dow Corning 5200 Formulation Aid.

Eine erfindungsgemäß besonders bevorzugte Gruppe von Wasser-in-Öl-Emulgatoren sind die Poly-(C₂-C₃)alkylenglycol-modifizierten Silicone, deren frühere INCI-Bezeichnung Dimethicone Copolyol lautete, bevorzugt Poly-(C₂-C₃)alkylenglycol-modifizierte Silicone mit den INCI-Bezeichnungen PEG-x Dimethicone mit x = 2 - 20, bevorzugt 3 - 17, besonders bevorzugt 11 - 12, Bis-PEG-y Dimethicone mit y = 3 - 25, bevorzugt 4 - 20, PEG/PPG a/b Dimethicone, wobei a und b unabhängig voneinander für Zahlen von 2 - 30, bevorzugt 3 - 24, besonders bevorzugt 12 - 20, insbesondere 14 - 18, stehen, Bis-PEG/PPG-c/d Dimethicone, wobei c und d unabhängig voneinander für Zahlen von 10 - 25, bevorzugt 14 - 20 und besonders bevorzugt 14 - 16, stehen, und Bis-PEG/PPG-e/f PEG/PPG g/h Dimethicone, wobei e, f, g und h unabhängig voneinander für Zahlen von 10 - 20, bevorzugt 14 - 18 und besonders bevorzugt 16, stehen, mit der Bedingung, dass diese ethoxylierten und/oder propoxylierten Siliconemulgatoren unter Normalbedingungen einen HLB-Wert im Bereich von 1,5 bis 6,5, bevorzugt 4 bis 6,5, aufweisen.

Zur Klarstellung sei angemerkt, dass diese vorgenannten bevorzugten Wasser-in-Öl-Emulgatoren b) nur mit Methylgruppen alkyliert sind und - wie die Bezeichnungen "Dimethicone Copolyol" und "PEG/PPG Dimethicone" bereits implizieren - keine höheren Alkylgruppen, z. B. Ethyl, Lauryl, Cetyl, im Molekül aufweisen.

Ein erfindungsgemäß außerordentlich bevorzugter Wasser-in-Öl-Emulgator ist PEG/PPG-18/18 Dimethicone, das einen HLB-Wert im Bereich von 4 bis 6,5 aufweist. Dieser besonders bevorzugte Wasser-in-Öl-Emulgator PEG/PPG-18/18 Dimethicone ist im Handel üblicherweise nicht als Reinsubstanz, sondern in einem kosmetischen Öl dispergiert erhältlich. Bevorzugte erfindungsgemäße Produkte umfassen eine Wasser-in-Öl-Emulsion, enthaltend den Wasser-in-Öl-Emulgator PEG/PPG-18/18 Dimethicone als Dispersion in Dimethicone, das eine Viskosität von 3 - 100 cSt, bevorzugt 5 - 50 cSt, besonders bevorzugt 10 - 20 cSt (jeweils bei 25°C), aufweist. Ein erfindungsgemäß besonders bevorzugtes Handelsprodukt ist Dow Corning ES-5227 DM von der Firma Dow Corning.

Wasser-in-Öl-Emulsionen, bei denen der Wasser-in-Öl-Emulgator b) nur ausgewählt ist aus PEG/PPG-18/18 Dimethicone, das einen HLB-Wert im Bereich von 4 bis 6,5 aufweist und das in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion, enthalten ist, sind erfindungsgemäß besonders bevorzugt.

Ein bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz in einer Gesamtmenge von 5 - 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion,
   b) mindestens einen Wasser-in-Öl-Emulgator mit einem HLB-Wert im Bereich von 1,5 bis 6,5, ausgewählt aus der Gruppe der PEG/PPG a/b Dimethicone, wobei a und b unabhängig voneinander für Zahlen von 2 - 30, bevorzugt 3 - 24, besonders bevorzugt 12 - 20, insbesondere 14 - 18, stehen, in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
   d) mindestens ein kosmetisches Öl, das kein Riechstoff und kein ätherisches Öl ist,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat
- mindestens einem Treibmittel,
- einer Aerosol Abgabevorrichtung.

Ein besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz in einer Gesamtmenge von 5 - 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion,
   b) mindestens einen Wasser-in-Öl-Emulgator mit einem HLB-Wert im Bereich von 1,5 bis 6,5, der nur ausgewählt ist aus PEG/PPG-18/18 Dimethicone, das einen HLB-Wert im Bereich von 4 bis 6,5 aufweist und das in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion, enthalten ist,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
   d) mindestens ein kosmetisches Öl, das kein Riechstoff und kein ätherisches Öl ist,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat
- mindestens einem Treibmittel,
- einer Aerosol Abgabevorrichtung.

Die erfindungsgemäßen Wasser-in-Öl-Emulsionen enthalten als dritten wesentlichen Bestandteil mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17.

Für das angestrebte Gleichgewicht zwischen der Emulsionsstabilität der erfindungsgemäßen Wasser-in-Öl-Emulsionen und der möglichst schnellen Wirkstofffreisetzung des schweißhemmenden Wirkstoffs aus der inneren Phase der Emulsion hat sich der Zusatz eines solchen Öl-in-Wasser-Emulgators oder Lösungsvermittlers mit einem HLB-Wert im Bereich von > 7 bis maximal 20 als überraschend vorteilhaft erwiesen.

Bevorzugte erfindungsgemäße Wasser-in-Öl-Emulsionen sind dadurch gekennzeichnet, dass der mindestens eine Öl-in-Wasser-Emulgator oder Lösungsvermittler c) einen HLB-Wert > 9, bevorzugt einen HLB-Wert > 12 bis 19 und insbesondere einen HLB-Wert > 14 bis 18 aufweist. Besonders bevorzugte Öl-in-Wasser-Emulgatoren oder Lösungsvermittler c) weisen einen HLB-Wert von 15 bis 17 auf.

Die Ermittlung des HLB-Wertes erfolgt erfindungsgemäß nach Griffin gemäß der Formel: HLB = 20 * (Mₕ / M), wobei Mₕ die Molmasse des hydrophilen Anteils eines Moleküls ist und M die Molmasse des gesamten Moleküls. Es ergibt sich damit eine Skala von 0 bis 20.

Bevorzugte erfindungsgemäße Wasser-in-Öl-Emulsionen sind dadurch gekennzeichnet, dass der mindestens eine Öl-in-Wasser-Emulgator oder Lösungsvermittler c) ausgewählt ist aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und verestert mit 1 bis 4 gesättigten oder ungesättigten, linearen oder verzweigten, gegebenenfalls hydroxylierten C₈ - C₃₀-Fettsäureresten, sofern diese Polyglycerinpartialester einen HLB-Wert im Bereich von > 7 bis 20 aufweisen, sowie Mischungen der vorgenannten Substanzen.

Die ethoxylierten C₈-C₂₄-Alkanole haben die Formel R¹O(CH₂CH₂O)ₙH, wobei R¹ für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid steht.

Erfindungsgemäß besonders bevorzugt sind Adduktverbindungen von 10 - 100, bevorzugt 10 - 30 Mol Ethylenoxid, an 1 Mol Alkanol, ausgewählt aus Caprylalkohol, 2-Ethylhexylalkohol, Caprinalkohol, Laurylalkohol, Isotridecylalkohol, Myristylalkohol, Cetylalkohol, Palmitoleylalkohol, Isocetylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid, bevorzugt 10 - 30 Mol Ethylenoxid, an 1 Mol Alkanol, ausgewählt aus technischen Fettalkoholen mit 12 - 18 Kohlenstoffatomen, wie beispielsweise Kokos-, Palm-, Palmkern- oder Talgfettalkohol, sind erfindungsgemäß bevorzugt. Außerordentlich bevorzugt ist Isoceteth-20. Die vorgenannten ethoxylierten Alkanole sind nichtionisch und werden auch als Polyethylenglycolether oder nichtionische Polyethylenglycolether bezeichnet.

Die ethoxylierten C₈-C₂₄-Carbonsäuren haben die Formel R¹OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 50 Mol Ethylenoxid, an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10-100, vorzugsweise 10 - 50, Mol Ethylenoxid an 1 Mol Säure, ausgewählt aus technischen Fettsäuren mit 12 - 18 Kohlenstoffatomen, wie Kokos-, Palm-, Palmkern- oder Talgfettsäure, sind bevorzugt. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt sind die C₁₂-C₁₈-Alkanole mit 10 - 30 Einheiten Ethylenoxid pro Molekül sowie Mischungen dieser Substanzen. Außerordentlich bevorzugt sind die C₁₄-C₁₈-Alkanole mit 20 - 30 Einheiten Ethylenoxid pro Molekül sowie Mischungen dieser Substanzen. Außerordentlich bevorzugt sind insbesondere Isoceteth-12, Isoceteth-20, Isoceteth-30, Isosteareth-12, Isosteareth-20, Isosteareth-30, Laureth-12 und Beheneth-20.

Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, bevorzugt 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2 liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Als Öl-in-Wasser-Emulgatoren geeignete ethoxylierte Sterine sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert im Bereich von > 7 bis 20 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Bevorzugt ist der mindestens eine Öl-in-Wasser-Emulgator oder Lösungsvermittler mit einem HLB-Wert im Bereich von > 7 bis 20 ausgewählt aus nichtionischen Polyethylenglycolethern mit einem entsprechenden HLB-Wert, das heißt, mit einem HLB-Wert im Bereich von > 7 bis 20. Erfindungsgemäß besonders bevorzugte Produkte enthalten mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf das Gewicht der gesamten treibmittelfreien Wasser-in-Öl-Emulsion. Weitere besonders bevorzugte Produkte enthalten mindestens einen nichtionischen Polyethylenglycolether mit einem HLB-Wert im Bereich von > 7 - 20, bevorzugt im Bereich von > 12 - 19, besonders bevorzugt im Bereich von > 14 - 18 und außerordentlich bevorzugt im Bereich von 15 bis 17, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion. Weitere besonders bevorzugte Produkte enthalten Isoceteth-20 in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion.

Ein bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20,
   d) mindestens ein kosmetisches Öl, das kein Riechstoff und kein ätherisches Öl ist,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat,
- mindestens einem Treibmittel,
- einer Aerosol Abgabevorrichtung.

Ein besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist,
   e) 5-Methyl-2-(1-methylethyl)cyclohexyl-N-ethyloxamat, bevorzugt (1R,2S,5R)-5-Methyl-2-(1 -methylethyl)cyclohexyl-N-ethyloxamat,
- mindestens einem Treibmittel,
- einer Aerosol Abgabevorrichtung.

Ein weiterer besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz in einer Gesamtmenge von 5 - 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion,
   b) mindestens einen Wasser-in-Öl-Emulgator mit einem HLB-Wert im Bereich von 1,5 - 6,5 in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist,
   e) 5-Methyl-2-(1-methylethyl)cyclohexyl-N-ethyloxamat, bevorzugt (1R,2S,5R)-5-Methyl-2-(1 -methylethyl)cyclohexyl-N-ethyloxamat,
- mindestens einem Treibmittel,
- einer Aerosol Abgabevorrichtung.

Die erfindungsgemäßen Wasser-in-Öl-Emulsionen enthalten als vierten wesentlichen Bestandteil mindestens ein kosmetisches Öl, das kein Riechstoff und kein ätherisches Öl ist. Das kosmetische Öl ist unter Normalbedingungen flüssig. Unter ätherischen Ölen werden erfindungsgemäß Gemische aus flüchtigen Komponenten verstanden, die durch Wasserdampfdestillation aus pflanzlichen Rohstoffen hergestellt werden, wie beispielsweise Citrusöle.

Erfindungsgemäß bevorzugte Produkte enthalten mindestens ein kosmetisches Öl, das kein Riechstoff und kein ätherisches Öl ist, in einer Gesamtmenge von 1 - 30 Gew.-%, bevorzugt 5-26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 10 - 12000 Pa (0,1 - 90 mm Hg), besonders bevorzugt 13 - 3000 Pa, außerordentlich bevorzugt 15 - 500 Pa, aufweisen.

Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.

Cyclomethicone sind im Stand der Technik als gut geeignete Öle für kosmetische Produkte, insbesondere für schweißhemmende und deodorierende Produkte, wie Sprays und Stifte, bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen Produkte maximal 1 Gew.-%, bevorzugt maximal 0,1 Gew.-%, Cyclomethicone, jeweils bezogen auf das Gewicht der Wasser-in-Öl-Emulsion, ohne das Gewicht des Treibmittels zu berücksichtigen.

Ein erfindungsgemäß bevorzugter Cyclomethicone-Ersatzstoff ist eine Mischung aus C₁₃-C₁₆-Iso-paraffinen, C₁₂ - C₁ₐ-Isoparaffinen und C₁₃ - C₁₅-Alkanen, deren Viskosität bei 25°C im Bereich von 2 bis 6 mPas liegt und die einen Dampfdruck bei 20°C im Bereich von 10 bis 150 Pa, bevorzugt 100 bis 150 Pa, aufweist. Eine solche Mischung ist z. B. unter der Bezeichnung SiClone SR-5 von der Firma Presperse Inc. erhältlich.

Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning^{®} 200 (0,65 cSt) und Dow Corning^{®} 200 (1,5 cSt) von Dow Corning enthalten sind, und niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.

Bevorzugte erfindungsgemäße Antitranspirant-Zusammensetzungen enthalten wegen des trockeneren Hautgefühls und der schnelleren Wirkstofffreisetzung mindestens ein flüchtiges Siliconöl, das cyclisch oder linear sein kann.

Weitere bevorzugte erfindungsgemäße Produkte enthalten wegen des trockeneren Hautgefühls und der schnelleren Wirkstofffreisetzung mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa. Dieses mindestens eine C₈-C₁₆-Isoparaffin ist bevorzugt in einer Gesamtmenge von 1 - 30 Gew.-%, bevorzugt 3 - 26 Gew.-%, besonders bevorzugt 5 - 24 Gew.-%, außerordentlich bevorzugt 8 - 17 Gew.-%, enthalten, jeweils bezogen auf das Gewicht der Wasser-in-Öl-Emulsion, ohne das Gewicht des Treibmittels zu berücksichtigen.

Weitere erfindungsgemäß bevorzugte Wasser-in-Öl-Emulsionen enthalten mindestens ein nichtflüchtiges kosmetisches Öl, ausgewählt aus nichtflüchtigen Siliconölen und nichtflüchtigen Nichtsiliconölen. Rückstände des gelösten Antitranspirantwirkstoffes, aber auch Rückstände von in der Emulsion unlöslichen Bestandteilen, wie Talkum oder Silicapartikel, können erfolgreich mit einem nichtflüchtigen Öl maskiert werden. Außerdem können mit einem Gemisch aus verschiedenen Ölen, insbesondere aus nichtflüchtigem und flüchtigem Öl, Parameter wie Hautgefühl, Sichtbarkeit des Rückstands und Stabilität der erfindungsgemäßen Zusammensetzung feinreguliert und besser an die Bedürfnisse der Verbraucher angepasst werden.

Erfindungsgemäß bevorzugte Produkte sind dadurch gekennzeichnet, dass das kosmetische Öl d) mindestens ein flüchtiges Öl mit einem Dampfdruck von 10 - 3000 Pa bei 20°C in einer Gesamtmenge von 10 - 100 Gew.-%, besonders bevorzugt 30 - 80 Gew.-%, jeweils bezogen auf das Gesamtgewicht der kosmetischen Öle d), umfasst.

Selbstverständlich ist es ebenfalls möglich, erfindungsgemäße Produkte mit einem geringen Anteil an flüchtigen Ölen - das heißt, mit 0,5 - 15 Gew.-% an flüchtigen Ölen, bezogen auf das Gewicht der treibmittelfreien Wasser-in-Öl-Emulsion - oder sogar ohne flüchtige Öle zu formulieren.

Als kosmetisches Öl d) erfindungsgemäß weiterhin besonders bevorzugt sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyl-octansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat, Ethylenglycoldipalmitat. n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv^{®} TN (C₁₂-C₁₅-Alkylbenzoat), sowie Benzoesäureisostearylester, z. B. erhältlich als Finsolv^{®} SB, 2-Ethylhexylbenzoat, z. B. erhältlich als Finsolv^{®} EB, und Benzoesäure-2-octyldode-cylester, z. B. erhältlich als Finsolv^{®} BOD.

Als für die Emulsionsstabilität und Wirkstofffreisetzung besonders vorteilhaft hat sich der Einsatz von Isopropylestern von C₁₂-C₁₈-Carbonsäuren, insbesondere der Einsatz von Isopropylmyristat, und besonders bevorzugt Mischungen von Isopropylmyristat mit C₁₀-C₁₃-Isoparaffin-Mischungen, letztere bevorzugt mit einem Dampfdruck bei 20°C von 10 - 400 Pa, erwiesen.

Ein weiteres besonders bevorzugtes Esteröl ist Triethylcitrat. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan sowie Mischungen dieser C₈-C₁₆-Isoparaffine. Weitere erfindungsgemäß bevorzugte Produkte enthalten Triethylcitrat und eine Mischung aus Isodecan, Isoundecan, Isododecan und Isotridecan.

Erfindungsgemäß bevorzugte Wasser-in-Öl-Emulsionen enthalten mindestens einen Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, in einer Gesamtmenge von 1 - 30 Gew.-%, bevorzugt 5 - 26 Gew.-%, besonders bevorzugt 9 - 24 Gew.-%, außerordentlich bevorzugt 12 - 17 Gew.-%, jeweils bezogen auf das Gewicht der gesamten treibmittelfreien Wasser-in-Öl-Emulsion.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllaurat.

Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol^{®} 318 oder Myritol^{®} 331 (BASF/ Cognis) mit unverzweigten Fettsäureresten sowie Glyceryltriisostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in dem erfindungsgemäßen Produkt aus. Besonders bevorzugt beträgt das Gesamtgewicht an Triglyceridölen 0,5 - 10 Gew.-%, bevorzugt 1 - 5 Gew.-%, jeweils bezogen auf das Gewicht der gesamten Wasser-in-Öl-Emulsion, ohne das Gewicht des Treibmittels zu berücksichtigen.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Di-n-caprylylcarbonat (Cetiol^{®} CC) oder Di-(2-ethylhexyl)carbonat (Tegosoft DEC). Ester der Kohlensäure mit C₁-C₅-Alkoholen, z. B. Glycerincarbonat oder Propylencarbonat, sind hingegen keine als kosmetisches Öl geeigneten Verbindungen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.-%, bevorzugt 1 - 5 Gew.-%, jeweils bezogen auf das Gewicht der gesamten Wasser-in-Öl-Emulsion, ohne das Gewicht des Treibmittels zu berücksichtigen.

Weitere kosmetische Öle, die erfindungsgemäß besonders bevorzugt sind, sind ausgewählt aus nichtflüchtigen Siliconölen. Erfindungsgemäß bevorzugte nichtflüchtige Siliconöle sind ausgewählt aus linearen Polyalkylsiloxanen mit einer kinematischen Viskosität bei 25°C von mindestens 5 cSt bis 2000 cSt, insbesondere ausgewählt aus linearen Polydimethylsiloxanen mit einer kinematischen Viskosität bei 25°C von 5 cSt bis 2000 cSt, bevorzugt 10 bis 350 cSt, besonders bevorzugt 50 - 100 cSt, wie sie z. B. unter den Handelsnamen Dow Corning^{®} 200 bzw. Xiameter PMX von Dow Corning bzw. Xiameter erhältlich sind. Weitere bevorzugte nichtflüchtige Siliconöle sind Phenyltrimethicone mit einer kinematischen Viskosität bei 25°C von 10 bis 100 cSt, bevorzugt von 15 - 30 cSt sowie Cetyldimethicone.

Erfindungsgemäß bevorzugte Wasser-in-Öl-Emulsionen enthalten mindestens ein nichtflüchtiges Siliconöl, das bevorzugt ausgewählt ist aus linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, in einer Gesamtmenge von 0,1 - 30 Gew.-%, bevorzugt 1 - 24 Gew.-%, besonders bevorzugt 2 - 18 Gew.-%, außerordentlich bevorzugt 4 - 10 Gew.-%, jeweils bezogen auf das Gewicht der gesamten treibmittelfreien Wasser-in-Öl-Emulsion.

Im Hinblick auf das angestrebte Gleichgewicht zwischen Emulsionsstabilität der erfindungsgemäßen Wasser-in-Öl-Emulsionen und der möglichst schnellen Freisetzung des schweißhemmenden Wirkstoffs aus der inneren Phase der Emulsion einerseits und der Stabilisierung des erfindungsgemäßen Kühlwirkstoffs 5-Methyl-2-(1-methylethyl)cyclohexyl-N-ethyloxamat andererseits hat sich der Einsatz von kosmetischen Ölen d), die ausgewählt sind aus Mischungen von C₈-C₁₆-Isoparaffinen und Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen, Mischungen von C₈-C₁₆-Isoparaffinen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, sowie Mischungen von C₈-C₁₆-Isoparaffinen und Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, als überraschend vorteilhaft erwiesen.

Ein besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion,
   e) 5-Methyl-2-(1-methylethyl)cyclohexyl-N-ethyloxamat, bevorzugt (1R,2S,5R)-5-Methyl-2-(1 -methylethyl)cyclohexyl-N-ethyloxamat,
- mindestens einem Treibmittel,
- einer Aerosol-Abgabevorrichtung.

Ein weiterer bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
   d) mindestens ein kosmetisches Öl, das kein Riechstoff und kein ätherisches Öl ist und das ausgewählt ist aus C₈-C₁₆-Isoparaffinen, Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, sowie Mischungen der vorgenannten Öle,
   e) 5-Methyl-2-(1-methylethyl)cyclohexyl-N-ethyloxamat, bevorzugt (1R,2S,5R)-5-Methyl-2-(1 -methylethyl)cyclohexyl-N-ethyloxamat,
- mindestens einem Treibmittel,
- einer Aerosol-Abgabevorrichtung.

Ein besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist und das ausgewählt ist aus C₈-C₁₆-Isoparaffinen, Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, sowie Mischungen der vorgenannten Öle, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion,
   e) 5-Methyl-2-(1-methylethyl)cyclohexyl-N-ethyloxamat, bevorzugt (1R,2S,5R)-5-Methyl-2-(1 -methylethyl)cyclohexyl-N-ethyloxamat,
- mindestens einem Treibmittel,
- einer Aerosol-Abgabevorrichtung.

Ein weiterer besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz in einer Gesamtmenge von 5 - 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion,
   b) mindestens einen Wasser-in-Öl-Emulgator mit einem HLB-Wert im Bereich von 1,5 - 6,5 in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist und das ausgewählt ist aus Mischungen von C₈-C₁₆-Isoparaffinen und Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt bis 2000 cSt, bevorzugt 10 bis 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion,
   e) 5-Methyl-2-(1-methylethyl)cyclohexyl-N-ethyloxamat, bevorzugt (1R,2S,5R)-5-Methyl-2-(1 -methylethyl)cyclohexyl-N-ethyloxamat,
- mindestens einem Treibmittel,
- einer Aerosol-Abgabevorrichtung.

Weiterhin war es möglich, den Cyclomethicone-Gehalt erfindungsgemäßer Produkte ohne Verschlechterung der Produkteigenschaften abzusenken. Bevorzugte erfindungsgemäße Produkte enthaltend daher, bezogen auf die gesamte treibmittelfreie Wasser-in-Öl-Emulsion, weniger als 5,0 Gew.-% Cyclomethicone, bevorzugt weniger als 3,0 Gew.-% Cyclomethicone und besonders bevorzugt weniger als 1,0 Gew.-% Cyclomethicone. Ganz besonders bevorzugte erfindungsgemäße Produkte sind frei von Cyclomethiconen.

Die erfindungsgemäßen Produkte enthalten als fünften wesentlichen Bestandteil den Kühlwirkstoff 5-Methyl-2-(1-methylethyl)cyclohexyl-N-ethyloxamat. Bevorzugt ist das Isomer (1R,2S,5R)-5-Methyl-2-(1-methylethyl)cyclohexyl-N-ethyloxamat enthalten. Dieser Kühlwirkstoff verbindet eine lang anhaltende kühlende Wirkung schon in niedrigen Einsatzkonzentrationen mit einem geringen Eigengeruch, einer hohen Hautverträglichkeit und einer guten Freisetzung insbesondere aus einer Wasser-in-Öl-Emulsion. Bevorzugte erfindungsgemäße Produkte enthalten 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, enthalten ist, wobei sich alle Mengenangaben auf das Gewicht der Wasser-in-Öl-Emulsion beziehen, ohne das Gewicht des Treibmittels zu berücksichtigen.

Ein weiterer bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
   d) mindestens ein kosmetisches Öl, das kein Riechstoff und kein ätherisches Öl ist und das ausgewählt ist aus C₈-C₁₆-Isoparaffinen, Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, sowie Mischungen der vorgenannten Öle,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, enthalten ist, wobei sich alle Mengenangaben auf das Gewicht der Wasser-in-Öl-Emulsion beziehen, ohne das Gewicht des Treibmittels zu berücksichtigen,
- mindestens einem Treibmittel,
- einer Aerosol-Abgabevorrichtung.

Ein besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist und das ausgewählt ist aus C₈-C₁₆-Isoparaffinen, Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, sowie Mischungen der vorgenannten Öle, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, enthalten ist, wobei sich alle Mengenangaben auf das Gewicht der Wasser-in-Öl-Emulsion beziehen, ohne das Gewicht des Treibmittels zu berücksichtigen,
- mindestens einem Treibmittel,
- einer Aerosol-Abgabevorrichtung.

Ein weiterer besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz in einer Gesamtmenge von 5 - 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion,
   b) mindestens einen Wasser-in-Öl-Emulgator mit einem HLB-Wert im Bereich von 1,5 - 6,5 in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist und das ausgewählt ist aus Mischungen von C₈-C₁₆-Isoparaffinen und Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt bis 2000 cSt, bevorzugt 10 bis 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, enthalten ist, wobei sich alle Mengenangaben auf das Gewicht der Wasser-in-Öl-Emulsion beziehen, ohne das Gewicht des Treibmittels zu berücksichtigen,
- mindestens einem Treibmittel,
- einer Aerosol-Abgabevorrichtung.

Bevorzugte erfindungsgemäße Produkte enthalten mindestens einen Riechstoff. Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Zur Parfümierung der erfindungsgemäßen Produkte können Parfüms, Parfümöle, Parfümölbestandteile oder einzelne Riechstoffverbindungen eingesetzt werden. Parfümöle bzw. Riechstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen z. B. Benzylethylether und Ambroxan , zu den Aldehyden z.B. die linearen Alkanale mit 8 - 18 C-Atomen, Citral, Citronellal, Citronellyloxyacetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol, alpha-Terpineol, beta-Terpineol, gamma-Terpineol, und delta-Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Riechstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Riechstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Haftfeste Riechstoffe, die im Rahmen der vorliegenden Erfindung einsetzbar sind, sind z. B. die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höher siedenden bzw. festen Riechstoffe natürlichen oder synthetischen Ursprungs können im Rahmen der vorliegenden Erfindung als haftfeste Riechstoffe bzw. Riechstoffgemische, also Riechstoffe, eingesetzt werden. Zu diesen Verbindungen zählen die nachfolgend genannten Verbindungen sowie Mischungen aus diesen: Ambrettolid, Allylacetat, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, beta-Naphtholethylether, beta-Naphtholmethylether, Nerol, Nitrobenzol, n-Nonylaldehyd, Nonylalkohol, n-Octylaldehyd, p-Oxy-Acetophenon, Pentadekanolid, beta-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, alpha-Terpineol, beta-Terpineol, gamma-Terpineol, delta-Terpineol, Thymen, Thymol, gamma-Undecalacton, Vanillin, Veratrumaldehyd, Zimtaldehyd, Zimtalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butandion, Limonen, Linalool, Linalylacetat, Linalylpropionat, Menthol, Menthon, Methyl-n-heptenon, Phellandren, Phenylacetaldehyd, Terpinylacetat, Zitral, Zitronellal.

Besonders bevorzugte erfindungsgemäße Produkte enthalten mindestens einen Riechstoff in einer Gesamtmenge von 0,00001 bis 10 Gew.-%, bevorzugt 0,5 - 7 Gew.-%, besonders bevorzugt 2 - 5 Gew.-%, jeweils bezogen auf die gesamte treibmittelfreie Wasser-in-Öl-Emulsion.

In einer weiteren bevorzugten Ausführungsform enthalten die erfindungsgemäßen Wasser-in-Öl-Emulsionen mindestens ein Polyol, ausgewählt aus den mehrwertigen C₂ - C₉-Alkanolen mit 2 - 6 Hydroxylgruppen und Polyethylenglycolen mit 3 - 20 Ethylenoxid-Einheiten sowie Mischungen hiervon. Besonders bevorzugte erfindungsgemäße Wasser-in-Öl-Emulsionen enthalten mindestens ein mehrwertiges C₂ - C₉-Alkanol mit 2 - 6 Hydroxylgruppen und/oder mindestens ein Polyethylenglycol mit 3 - 20 Ethylenoxid-Einheiten.

Bevorzugte erfindungsgemäße Produkte sind dadurch gekennzeichnet, dass die Wasser-in-Öl-Emulsion weiterhin mindestens ein mehrwertiges C₂ - C₉-Alkanol mit 2 - 6 Hydroxylgruppen und/oder mindestens ein Polyethylenglycol mit 3 - 20 Ethylenoxid-Einheiten, ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan sowie Mischungen der vorgenannten Substanzen enthält. Diese Substanzen haben einen positiven Einfluss auf das Rückstandsverhalten, die Emulsionsstabilität und eine lagerstabile Sprühbarkeit der erfindungsgemäßen Wasser-in-Öl-Emulsionen. Ein diesbezüglich besonders bevorzugtes mehrwertiges C₂ - C₉-Alkanol mit 2 - 6 Hydroxylgruppen ist 1,2-Propylenglycol.

Der Gewichtsanteil des mehrwertigen Cm- C₉-Alkanols mit 2 - 6 Hydroxylgruppen und/oder des Polyethylenglycols mit 3 - 20 Ethylenoxid-Einheiten beträgt, bezogen auf den Gesamtgehalt dieser Substanzen in der treibmittelfreien Wasser-in-Öl-Emulsion, vorzugsweise 0,5 - 35 Gew.-%, bevorzugt 2,0 - 30 Gew.-%, besonders bevorzugt 5 - 25 Gew.-%, weiterhin bevorzugt 10 - 23 Gew.-% oder 12 - 18 Gew.-%.

Ein weiterer bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
   d) mindestens ein kosmetisches Öl, das kein Riechstoff und kein ätherisches Öl ist und das ausgewählt ist aus C₈-C₁₆-Isoparaffinen, Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, sowie Mischungen der vorgenannten Öle,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, wobei sich alle Mengenangaben auf das Gewicht der Wasser-in-Öl-Emulsion beziehen, ohne das Gewicht des Treibmittels zu berücksichtigen,
   f) mindestens ein mehrwertiges C₂ - C₉-Alkanol mit 2 - 6 Hydroxylgruppen und/oder mindestens ein Polyethylenglycol mit 3 - 20 Ethylenoxid-Einheiten, bevorzugt ausgewählt aus 1,2-Propylenglycol, in einer Gesamtmenge von 0,5 - 35 Gew.-%, bevorzugt 2,0 - 30 Gew.-%, besonders bevorzugt 5 - 25 Gew.-%, weiterhin bevorzugt 10 - 23 Gew.-% oder 12 - 18 Gew.-%, wobei sich alle Mengenangaben auf das Gewicht der Wasser-in-Öl-Emulsion beziehen, ohne das Gewicht des Treibmittels zu berücksichtigen,
- mindestens einem Treibmittel,
- einer Aerosol-Abgabevorrichtung.

Ein besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz,
   b) mindestens einen Wasser-in-Öl-Emulgator mit einem HLB-Wert im Bereich von 1,5 - 6,5,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist und das ausgewählt ist aus C₈-C₁₆-Isoparaffinen, Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, sowie Mischungen der vorgenannten Öle, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, wobei sich alle Mengenangaben auf das Gewicht der Wasser-in-Öl-Emulsion beziehen, ohne das Gewicht des Treibmittels zu berücksichtigen,
   f) mindestens ein mehrwertiges C₂ - C₉-Alkanol mit 2 - 6 Hydroxylgruppen und/oder mindestens ein Polyethylenglycol mit 3 - 20 Ethylenoxid-Einheiten, bevorzugt ausgewählt aus 1,2-Propylenglycol, in einer Gesamtmenge von 0,5 - 35 Gew.-%, bevorzugt 2,0 - 30 Gew.-%, besonders bevorzugt 5 - 25 Gew.-%, weiterhin bevorzugt 10 - 23 Gew.-% oder 12 - 18 Gew.-%, wobei sich alle Mengenangaben auf das Gewicht der Wasser-in-Öl-Emulsion beziehen, ohne das Gewicht des Treibmittels zu berücksichtigen,
- mindestens einem Treibmittel,
- einer Aerosol-Abgabevorrichtung.

Ein weiterer besonders bevorzugter Gegenstand der vorliegenden Anmeldung ist ein kosmetisches Produkt, bestehend aus
- einer Wasser-in-Öl-Emulsion, enthaltend
   a) mindestens ein schweißhemmendes Aluminiumsalz in einer Gesamtmenge von 5 - 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion,
   b) mindestens einen Wasser-in-Öl-Emulgator mit einem HLB-Wert im Bereich von 1,5 - 6,5 in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion,
   c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt aus nichtionischen Polyethylenglycolethern, insbesondere Isoceteth-20, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion,
   d) mindestens ein kosmetisches Öl, das kein Riechstoff und kein ätherisches Öl ist und das ausgewählt ist aus Mischungen von C₈-C₁₆-Isoparaffinen und Estern der linearen oder verzweigten gesättigten oder ungesättigten Fettalkohole mit 2 - 30 Kohlenstoffatomen mit linearen oder verzweigten gesättigten oder ungesättigten, optional hydroxylierten Fettsäuren mit 2 - 30 Kohlenstoffatomen und linearen Polyalkylsiloxanen mit einer kinematischen Viskosität bei 25°C von 5 cSt bis 2000 cSt, bevorzugt 10 bis 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion,
   e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, wobei sich alle Mengenangaben auf das Gewicht der Wasser-in-Öl-Emulsion beziehen, ohne das Gewicht des Treibmittels zu berücksichtigen,
   f) mindestens ein mehrwertiges C₂ - C₉-Alkanol mit 2 - 6 Hydroxylgruppen und/oder mindestens ein Polyethylenglycol mit 3 - 20 Ethylenoxid-Einheiten, bevorzugt ausgewählt aus 1,2-Propylenglycol, in einer Gesamtmenge von 0,5 - 35 Gew.-%, bevorzugt 2,0 - 30 Gew.-%, besonders bevorzugt 5 - 25 Gew.-%, weiterhin bevorzugt 10 - 23 Gew.-% oder 12 - 18 Gew.-%, wobei sich alle Mengenangaben auf das Gewicht der Wasser-in-Öl-Emulsion beziehen, ohne das Gewicht des Treibmittels zu berücksichtigen,
- mindestens einem Treibmittel,
- einer Aerosol-Abgabevorrichtung.

Auf Grundlage der erfindungsgemäßen Kombinationen ist es möglich, den Ethanol-Gehalt erfindungsgemäßer Produkte ohne Verschlechterung der Produkteigenschaften abzusenken. Bevorzugte erfindungsgemäße Produkte enthaltend daher, bezogen auf die gesamte treibmittelfreie Wasser-in-Öl-Emulsion, weniger als 4,0 Gew.-% Ethanol, vorzugsweise weniger als 3,0 Gew.-% Ethanol und besonders bevorzugt weniger als 1,0 Gew.-% Ethanol. Ganz besonders bevorzugte erfindungsgemäße Produkte sind frei von Ethanol.

Die erfindungsgemäßen Produkte enthalten Wasser bevorzugt in einer Gesamtmenge von 5 - 55 Gew.-%, bevorzugt 10 - 40 Gew.-%, besonders bevorzugt 15 - 30 Gew.-%, außerordentlich bevorzugt 20 - 25 Gew.-%, jeweils bezogen auf das Gewicht der gesamten treibmittelfreien Wasser-in-Öl-Emulsion.

Um den Kühleffekt der erfindungsgemäßen Produkte weiter zu optimieren und zum Beispiel kurz nach Applikation des Produkts auf die Haut einen sehr starken Effekt ("Flash") zu erzielen, der dann durch 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, weiter verlängert wird, oder auch um den Kühleffekt von einem frischen Geruch begleiten zu lassen, ist es bevorzugt, mindestens einen weiteren Kühlwirkstoff zuzusetzen

Weitere erfindungsgemäße Produkte enthalten zusätzlich zu 5-Methyl-2-(1-methylethyl)cyclohexyl-N-ethyloxamat mindestens einen weiteren Kühlwirkstoff, der ausgewählt aus L-Menthol, D-Menthol und DL-Menthol, bevorzugt L-Menthol, weiterhin aus Isopulegol, insbesondere (-)-Isopulegol (FEMA 2962), N-2,3-Trimethyl-2-Isopropylbutamid, Menthyllactat, Menthylpyrrolidoncarbonsäure, Menthylmethylether, Menthoxypropan-1,2-diol, Menthonglycerinacetal (9-Methyl-6-(1-methylethyl)-1,4-dioxaspiro(4.5)decan-2-methanol), Monomenthylsuccinat, 2-Hydroxymethyl-3,5,5-trimethylcyclohexanol, 2-Isopropyl-N,2,3-trimethylbutyramid (FEMA 3804), N-Ethyl-p-menthan-3-carboxamid (FEMA 3455), insbesondere 1R,3R,4S-N-Ethyl-p-menthane-3-carboxamid, Ethyl-3-(p-menthan-3-carboxamido)acetat (FEMA 4309), (1R,2S,5R)-N-(4-Methoxyphenyl)-p-menthancarboxamid (FEMA 4681), N-Ethyl-2,2-diisopropylbutanamid (FEMA 4557), N-Cyclopropyl-5-methyl-2-propan-2-ylcyclohexan-1-carboxamid (FEMA 4693), N-(4-Cyanomethylphenyl)-p-menthancarboxamid (FEMA 4496), N-(2-(Pyridin-2-yl)ethyl)-3-p-menthancarboxamid (FEMA 4549), N-(2-Hydroxyethyl)-2-isopropyl-2,3-dimethylbutanamid (FEMA4602), N-(1,1-Dimethyl-2-hydroxyethyl)-2,2-diethylbutanamid (FEMA 4603), (2S,5R)-N-[4-(2-Amino-2-oxoethyl)phenyl]-p-menthancarboxamid (FEMA 4684), 2-[(2-p-Menthoxy)ethoxy]ethanol (FEMA 4718), (2,6-Diethyl-5-isopropyl-2-methyltetrahydropyran (FEMA 4680), 3-(I-Menthoxy)-2-methylpropan-1,2-diol (FEMA 3849), p-Menthan-3,8-diol, insbesondere (+)-cis-p-Menthan-3,8-diol und (-)-trans-p-Menthan-3,8-diol sowie Mischungen aus (+)-cis-p-Menthan-3,8-diol und (-)-trans-p-Menthan-3,8-diol, insbesondere eine Mischung im Gewichtsverhältnis 62:38 (FEMA 4053), (1R,3R,4S)-3-Menthyl-3,6-dioxaheptanoat, (1 R,2S,5R)-3-Menthylmethoxyacetat, (1 R,2S,5R)-3-Menthyl-3,6,9-trioxadecanoat, (1R,2S,5R)-3-Menthyl-3,6,9-trioxadecanoat, (1R,2S,5R)-3-Menthyl-(2-hydroxyethoxy)-acetat, (1R,2S,5R)-Menthyl-11-hydroxy-3,6,9-trioxaundecanoat, (-)-Cubebol (FEMA 4497), N-(4-Cyanomethylphenyl)-p-menthancarboxamid, N,N-Dimethylmenthylsuccinamid (2-Isopropyl-5-methylcyclohexyl-4-(dimethylamino)-4-oxobutanoat, FEMA 4230), 6-Isopropyl-3,9-dimethyl-1,4-dioxaspiro[4.5]decan-2-on (FEMA 4285), sowie Mischungen dieser Substanzen, wobei L-Menthol besonders bevorzugt ist.

Erfindungsgemäß bevorzugte Produkte sind dadurch gekennzeichnet, dass das Menthol ausgewählt ist aus L-Menthol, D-Menthol und DL-Menthol, bevorzugt ausgewählt aus DL-Menthol. Weitere erfindungsgemäß bevorzugte Produkte enthalten 0,09 - 5 Gew.-%, bevorzugt 0,1 - 2,5 Gew.-%, besonders bevorzugt 0,2 - 1,8 Gew.-%, außerordentlich bevorzugt 0,3 - 1,0 Gew.-%, Menthol, ausgewählt aus L-Menthol, D-Menthol und DL-Menthol, wobei sich die Gew.-%-Angaben auf das Gesamtgewicht der Wasser-in-Öl-Emulsion beziehen, ohne ggf. vorhandenes Treibmittel zu berücksichtigen.

Weitere erfindungsgemäß bevorzugte Produkte enthalten insgesamt 0,05 - 3 Gew.-%, bevorzugt 0,1 - 2,5 Gew.-%, besonders bevorzugt 0,2 - 1,8 Gew.-%, außerordentlich bevorzugt 0,3 - 1,0 Gew.-%, von einem oder mehreren weiteren Kühlwirkstoffen, der ausgewählt ist aus Isopulegol, insbesondere (-)-Isopulegol (FEMA 2962), N-2,3-Trimethyl-2-Isopropylbutamid, Menthyllactat, Menthylpyrrolidoncarbonsäure, Menthylmethylether, Menthoxypropan-1,2-diol, Menthonglycerinacetal (9-Methyl-6-(1-methylethyl)-1,4-dioxaspiro(4.5)decan-2-methanol), Monomenthylsuccinat, 2-Hydroxymethyl-3,5,5-trimethylcyclohexanol, 2-Isopropyl-N,2,3-trimethylbutyramid (FEMA 3804), N-Ethyl-p-menthan-3-carboxamid (FEMA 3455), insbesondere 1 R,3R,4S-N-Ethyl-p-menthane-3-carboxamid, Ethyl-3-(p-menthan-3-carboxamido)acetat (FEMA 4309), (1R,2S,5R)-N-(4-Methoxyphenyl)-p-menthancarboxamid (FEMA 4681), N-Ethyl-2,2-diisopropylbutanamid (FEMA 4557), N-Cyclopropyl-5-methyl-2-propan-2-ylcyclohexan-1-carboxamid (FEMA 4693), N-(4-Cyanomethylphenyl)-p-menthancarboxamid (FEMA 4496), N-(2-(Pyridin-2-yl)ethyl)-3-p-menthancarboxamid (FEMA 4549), N-(2-Hydroxyethyl)-2-isopropyl-2,3-dimethylbutanamid (FEMA 4602), N-(1,1-Dimethyl-2-hydroxyethyl)-2,2-diethylbutanamid (FEMA 4603), (2S,5R)-N-[4-(2-Amino-2-oxoethyl)-phenyl]-p-menthancarboxamid (FEMA 4684), 2-[(2-p-Menthoxy)ethoxy]ethanol (FEMA 4718), (2,6-Diethyl-5-isopropyl-2-methyltetrahydropyran (FEMA 4680), 3-(I-Menthoxy)-2-methylpropan-1,2-diol (FEMA 3849), p-Menthan-3,8-diol, insbesondere (+)-cis-p-Menthan-3,8-diol und (-)-trans-p-Menthan-3,8-diol sowie Mischungen aus (+)-cis-p-Menthan-3,8-diol und (-)-trans-p-Menthan-3,8-diol, insbesondere eine Mischung im Gewichtsverhältnis 62:38 (FEMA 4053), (1R,3R,4S)-3-Menthyl-3,6-dioxaheptanoat, (1R,2S,5R)-3-Menthylmethoxyacetat, (1R,2S,5R)-3-Menthyl-3,6,9-trioxadecanoat, (1R,2S,5R)-3-Menthyl-3,6,9-trioxadecanoat, (1R,2S,5R)-3-Menthyl-(2-hydroxyethoxy)acetat, (1R,2S,5R)-Menthyl-11-hydroxy-3,6,9-trioxaundecanoat, (-)-Cubebol (FEMA 4497), N-(4-Cyanomethylphenyl)-p-menthancarboxamid, N,N-Dimethylmenthylsuccinamid (2-Isopropyl-5-methylcyclohexyl-4-(dimethylamino)-4-oxobutanoat, FEMA 4230), 6-Isopropyl-3,9-dimethyl-1,4-dioxaspiro[4.5]decan-2-on (FEMA 4285), sowie Mischungen dieser Substanzen, wobei sich die Gew.-%-Angaben auf das Gesamtgewicht der Wasser-in-Öl-Emulsion beziehen, ohne ggf. vorhandenes Treibmittel zu berücksichtigen.

Die erfindungsgemäßen Produkte enthalten bevorzugt ein oder mehrere Konservierungsmittel. Erfindungsgemäß bevorzugte Konservierungsmittel sind Formaldehydabspalter (wie z. B. 1,3-Dimethylol-4,4-dimethylhydantoin, INCI-Bezeichnung DMDM Hydantoin), lodopropinylbutylcarbamate wie 3-lod-2-propinylbutylcarbamat, Parabene (d. h. para-Hydroxybenzoesäurealkylester, wie Methyl-, Ethyl-, Propyl- und/oder Butylparaben), Phenoxyethanol, Ethanol, Benzoesäure, Dibromdicyanobutan (2-Brom-2-brommethylglutarodinitril), 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate. Erfindungsgemäß besonders bevorzugte Konservierungsmittel sind ausgewählt aus Parabenen (Methyl-, Ethyl-, Propyl- und/oder Butylparaben) und/ oder Phenoxyethanol.

Die Konservierungsmittel sind in den erfindungsgemäßen Produkten, bezogen auf die gesamte treibmittelfreie Wasser-in-Öl-Emulsion, vorzugsweise in einer Gesamtmenge von 0,01 - 3, bevorzugt 0,1 - 1,5 und besonders bevorzugt 0,2 - 1,0 Gew.-% enthalten.

In einer bevorzugten Ausführungsform enthalten die erfindungsgemäßen Produkte als deodorierenden Wirkstoff mindestens ein Silbersalz, das bevorzugt ausgewählt ist aus Silbersulfat, Silbernitrat, Silbercitrat, Silberdihydrogencitrat, Silberlactat, Silberacetat, Silbermalat, Silbersuccinat, Silbertartrat, Silbermandelat, Silbersalicylat, Silbergluconat, Silberadipat und Silbergalactarat, sowie aus Mischungen dieser Salze. Außerordentlich bevorzugt sind Silbersulfat, Silbercitrat, Silberdihydrogencitrat und Silberlactat, sowie Mischungen dieser Salze.

Weitere bevorzugte erfindungsgemäße Zusammensetzungen enthalten mindestens ein Silbersalz, das bevorzugt ausgewählt ist aus Silbersulfat, Silbernitrat, Silbercitrat, Silberdihydrogencitrat, Silberlactat, Silberacetat, Silbermalat, Silbersuccinat, Silbertartrat, Silbermandelat, Silbersalicylat, Silbergluconat, Silberadipat und Silbergalactarat, sowie aus Mischungen dieser Salze, in solchen Mengen, dass Silber in einer Gesamtmenge von 1 - 100 ppm, bevorzugt 2 - 50 ppm, besonders bevorzugt 5 - 20 ppm, außerordentlich bevorzugt 7 - 10 ppm, jeweils bezogen auf das Gewicht der treibmittelfreien Wasser-in-Öl-Emulsion, enthalten ist. Anhand der Molmassen von Silber (107,87 g/mol) und den jeweiligen Silbersalzen - Silberlactat z. B. hat eine Molmasse von 196,94 g/mol - kann die entsprechend nötige Menge an Silbersalz(en) berechnet werden.

In einer weiteren bevorzugten Ausführungsform enthalten die erfindungsgemäßen Produkte als deodorierenden Wirkstoff mindestens einen aromatischen Alkohol der Struktur (AA-1), 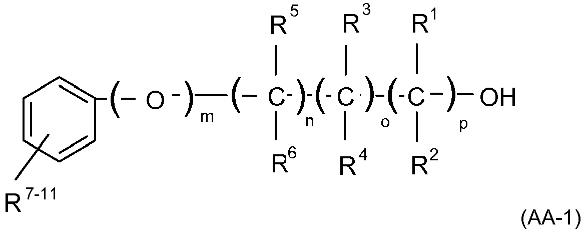 wobei
die Reste R¹ bis R⁶ unabhängig voneinander ein Wasserstoffatom, eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt und substituiert sein können mit OH-Gruppen oder Alkoxy-Gruppen mit 1 bis 5 Kohlenstoffatomen,
die Reste R⁷ bis R¹¹ unabhängig voneinander ein Wasserstoffatom, ein Halogenatom, insbesondere ein Chloratom, oder eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, insbesondere mit einer Methoyxgruppe,
m = 0 oder 1 ist, n, o, p = unabhängig voneinander ganze Zahlen von 0 bis 10 sind, wobei mindestens einer der Werte n, o, p ≠ 0 ist.

Besonders bevorzugte erfindungsgemäße Produkte enthalten mindestens einen Alkohol AA-1, wie vorstehend beschrieben, der ausgewählt ist aus Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1-ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethyl-propan-1-ol, 2-Allyl-3-phenylpropan-1-ol und 2-n-Pentyl-3-phenylpropan-1-ol sowie Mischungen hiervon. Außerordentlich bevorzugt ist 2-Benzylheptan-1-ol sowie Mischungen von 2-Benzylheptan-1-ol und Phenoxyethanol. Weitere besonders bevorzugte erfindungsgemäße Produkte enthalten mindestens einen Alkohol AA-1, wie vorstehend beschrieben, in einer Gesamtmenge von 0,05 - 10 Gew.-%, bevorzugt 0,1 - 5 Gew.-%, besonders bevorzugt 0,2 - 2 Gew.-%, außerordentlich bevorzugt 0,3 - 1,5 Gew.-%, jeweils bezogen auf das Gewicht der Wasser-in-Öl-Emulsion, ohne das Gewicht des Treibmittels zu berücksichtigen. Außerordentlich bevorzugte erfindungsgemäße Produkte enthalten 2-Benzylheptan-1-ol in einer Gesamtmenge von 0,05 - 1,5 Gew.-%, bevorzugt 0,1 - 1 Gew.-%, besonders bevorzugt 0,2 - 0,5 Gew.-%, jeweils bezogen auf das Gewicht der Wasser-in-Öl-Emulsion, ohne das Gewicht des Treibmittels zu berücksichtigen.

In einer weiteren bevorzugten Ausführungsform enthalten die erfindungsgemäßen Produkte als deodorierenden Wirkstoff mindestens ein 1,2-Alkandiol mit 5 bis 12 C-Atomen, das durch die Formel HO-CH₂-CH(OH)-(CH₂)ₙ-CH₃ beschrieben wird, in der n für die Zahlen 2, 3, 4, 5, 6, 7, 8 oder 9 steht. Besonders bevorzugte erfindungsgemäß verwendete 1,2-Alkandiole mit 5 bis 12 C-Atomen sind ausgewählt aus 1,2-Pentandiol, 1,2-Hexandiol, 1,2-Octandiol, 1,2-Decandiol und Mischungen hiervon. Eine ganz besonders bevorzugte erfindungsgemäße Kombination sind Mischungen aus 1,2-Hexandiol und 1,2-Octandiol, vorzugsweise im Gewichtsverhältnis 10:1 bis 1:10, weiter bevorzugt von 5:1 bis 1:5, besonders bevorzugt im Gewichtsverhältnis 1:1.

Bevorzugte erfindungsgemäße Produkte enthalten mindestens ein 1,2-Alkandiol mit 5 bis 12 C-Atomen, das durch die Formel HO-CH₂-CH(OH)-(CH₂)ₙ-CH₃ beschrieben wird, in der n für die Zahlen 2, 3, 4, 5, 6, 7, 8 oder 9 steht, in einer Gesamtmenge von 0,2 - 15 Gew.-%, bevorzugt 0,3 - 10 Gew.-%, besonders bevorzugt 0,4 - 5 Gew.-% und außerordentlich bevorzugt 0,5 - 2 Gew.-%, jeweils bezogen auf das Gewicht der Wasser-in-Öl-Emulsion, ohne das Gewicht des Treibmittels zu berücksichtigen. Außerordentlich bevorzugte erfindungsgemäße Produkte enthalten 0,2 - 0,5 Gew.-% 1,2-Hexandiol und 0,2 - 0,5 Gew.-% 1,2-Octandiol, jeweils bezogen auf das Gewicht der Wasser-in-Öl-Emulsion, ohne das Gewicht des Treibmittels zu berücksichtigen.

Weitere bevorzugte erfindungsgemäße Produkte sind gekennzeichnet durch einen Gehalt an dem deodorierenden Wirkstoff 3-(2-Ethylhexyloxy)-1,2-propandiol, bevorzugt in einer Gesamtmenge von 0,05 - 5 Gew.-%, bevorzugt 0,1 - 2 Gew.-%, besonders bevorzugt 0,2 - 1,5 Gew.-%, außerordentlich bevorzugt 0,5 - 1,0 Gew.-%, jeweils bezogen auf das Gewicht der Wasser-in-Öl-Emulsion, ohne das Gewicht des Treibmittels zu berücksichtigen.

Weitere bevorzugte erfindungsgemäße Produkte sind gekennzeichnet durch einen Gehalt an Tropolon (2-Hydroxy-2,4,6-Cycloheptatrienon), bevorzugt in einer Menge von 0,001 - 0,1 Gew.-%, bezogen auf das Gewicht der Wasser-in-Öl-Emulsion, ohne das Gewicht des Treibmittels zu berücksichtigen.

Weitere bevorzugte erfindungsgemäße Produkte sind gekennzeichnet durch einen Gehalt an dem deodorierenden Wirkstoff Triethylcitrat. Triethylcitrat ist ein bekannter Deodorantwirkstoff, der als Enzyminhibitor für Esterasen und Lipasen wirkt und somit zur Breitbandwirkung erfindungsgemäßer Produkte beiträgt. Bevorzugte erfindungsgemäße Produkte enthalten 0,5 - 15 Gew.-%, bevorzugt 3 - 8 Gew.-%, außerordentlich bevorzugt 4 - 6 Gew.-%, jeweils bezogen auf das Gewicht der Wasser-in-Öl-Emulsion, ohne das Gewicht des Treibmittels zu berücksichtigen.

Apokriner Schweiß stellt eine komplexe Mischung dar, die unter anderem Sebum und andere Fette sowie Steroide enthält. Steroide selbst sind nicht wasserlöslich. Um mit den Körperflüssigkeiten abtransportiert werden zu können, liegen sie normalerweise als Sulfat oder als Glucuronid vor. Auf der Haut erfolgt die Spaltung dieser Steroidester in die flüchtigen freien Steroide durch hydrolytische Enzyme der Hautbakterien, insbesondere der coryneformen Bakterien. Prinzipiell sind dazu alle bakteriellen Exoesterasen in der Lage, besonders aber die Enzyme Arylsulfatase und beta-Glucuronidase. Verbindungen, die Arylsulfatase oder beta-Glucuronidase inhibieren, stellen daher erfindungsgemäß bevorzugte Deodorantwirkstoffe dar.

Die Entwicklung der kurz- und mittelkettigen Fettsäuren, die wesentlich zum Körpergeruch beitragen, beginnt mit der Spaltung von Hautlipiden zu verzweigten langkettigen Fettsäuren. Die Spaltung der Hautlipide, die überwiegend als Glycerinester vorliegen, erfolgt im Wesentlichen durch Propionibacterium, Corynebacterium A und Staphylococcus-Spezies (A.G. James et al., Generation and Turnover of Volatile Fatty Acids by Axillary Bacteria, 22nd IFSCC Congress, Edinburgh, 2002, Poster 108). A.G. James et al. offenbaren weiterhin, dass aus den langkettigen verzweigten Fettsäuren durch die hydrolytischen Enzyme von einem bestimmten Corynebacterium, das A.G. James et al. als Corynebacterium A bezeichnen, sowohl kurzkettige C₂ - C₅-Fettsäuren als auch mittelkettige C₆ - C₁₂-Fettsäuren gebildet werden, die hauptsächlich für den axillaren Körpergeruch verantwortlich sind. Prinzipiell sind alle bakteriellen Exoesterasen zu dieser Lipidspaltung fähig, besonders aber das Enzym Lipase. Verbindungen, die Lipase inhibieren, stellen daher ebenfalls erfindungsgemäß bevorzugte Deodorantwirkstoffe dar.

Eine weitere Verbindungsklasse, die ebenfalls bei der bakteriellen Zersetzung der Schweißbestandteile entsteht und zum Körpergeruch beiträgt, sind gesättigte und ungesättigte Aldehyde, vor allem solche mit einer Kettenlänge von C₆ - C₁₂, insbesondere Hexanal, Heptanal, Octenal und Nonenal. Diese entstehen durch beta-Spaltung aus den Hydroperoxiden, die unter Einwirkung der 5-Lipoxigenase auf ungesättigte Fettsäuren gebildet werden. Verbindungen, die das Enzym 5-Lipoxigenase inhibieren, stellen daher ebenfalls erfindungsgemäß bevorzugte Deodorantwirkstoffe dar.

Weiterhin ist bekannt, dass stark übel riechende Komponenten des menschlichen Körpergeruchs und Mundgeruchs insbesondere durch enzymatische Reaktion freigesetzte flüchtige Schwefelverbindungen, so genannte "volatile sulfur compounds" (VSC), darstellen. Schwefelhaltige Verbindungen kommen als wasserlösliche Aminosäurekonjugate mit dem Schweiß auf die menschliche Haut. Dort werden sie von Hautbakterien (vor allem von Staphylokokken und Corynebakterien) durch enzymatische Reaktion freigesetzt. Ein Enzym, das bei der Freisetzung von VSCs eine besondere Rolle spielt, ist die Cystathionin-beta-Lyase. Dieses Enzym spaltet VSCs von den Aminosäuren ab und ist somit eine wichtige Ursache bei der Entstehung von Körpergeruch. Verbindungen, die das Enzym Cystathionin-beta-Lyase inhibieren, stellen daher ebenfalls erfindungsgemäß bevorzugte Deodorantwirkstoffe dar.

Weitere bevorzugte erfindungsgemäße Produkte sind gekennzeichnet durch einen Gehalt an mindestens einer Verbindung, die ein Inhibitor des Enzyms Arylsulfatase ist. Erfindungsgemäß bevorzugte Deodorantwirkstoffe, die als Arylsulfatase-Inhibitor wirken, sind solche, wie sie in US 5643559, US 5676937, WO2001/099376 A2, EP 1430879 A1 und DE 10216368 A1 offenbart sind. Weitere besonders bevorzugte erfindungsgemäße Produkte sind gekennzeichnet durch einen Gehalt an mindestens einer Verbindung, die ein Inhibitor des Enzyms Arylsulfatase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Wasser-in-Öl-Emulsion, ohne das Gewicht des Treibmittels zu berücksichtigen.

Weitere bevorzugte erfindungsgemäße Produkte sind gekennzeichnet durch einen Gehalt an mindestens einer Verbindung, die ein Inhibitor des Enzyms beta-Glucuronidase ist. Erfindungsgemäß bevorzugte Deodorantwirkstoffe, die als beta-Glucuronidase-Inhibitor wirken, sind solche, wie sie in WO2003/039505 A2 offenbart sind. Weitere besonders bevorzugte erfindungsgemäße Produkte sind gekennzeichnet durch einen Gehalt an mindestens einer Verbindung, die ein Inhibitor des Enzyms beta-Glucuronidase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Wasser-in-Öl-Emulsion, ohne das Gewicht des Treibmittels zu berücksichtigen.

Weitere bevorzugte erfindungsgemäße Produkte sind gekennzeichnet durch einen Gehalt an mindestens einer Verbindung, die ein Inhibitor des Enzyms Lipase ist. Erfindungsgemäß bevorzugte Deodorantwirkstoffe, die als Lipase-Inhibitor wirken, sind ausgewählt aus denen, die in EP 1428520 A2 offenbart sind, weiterhin ausgewählt aus den Aminomethylenmalonsäure-Derivaten gemäß DE 3018132 A1, den Ethylenoxid-Propylenoxid-Copolymeren gemäß GB 2335596 A1 und den Salzen der Phytinsäure gemäß EP 650 720 A1. Weitere besonders bevorzugte erfindungsgemäße Produkte sind gekennzeichnet durch einen Gehalt an mindestens einer Verbindung, die ein Inhibitor des Enzyms Lipase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Wasser-in-Öl-Emulsion, ohne das Gewicht des Treibmittels zu berücksichtigen.

Weitere besonders bevorzugte erfindungsgemäße Produkte sind gekennzeichnet durch einen Gehalt an mindestens einer Verbindung, die ein Inhibitor der 5-Lipoxigenase ist. Erfindungsgemäß bevorzugte Deodorantwirkstoffe, die als 5-Lipoxigenase-Inhibitor wirken, sind in EP 1428519 A2 offenbart. Weitere besonders bevorzugte erfindungsgemäße Produkte sind gekennzeichnet durch einen Gehalt an mindestens einer Verbindung, die ein Inhibitor des Enzyms 5-Lipoxigenase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Wasser-in-Öl-Emulsion, ohne das Gewicht des Treibmittels zu berücksichtigen.

Weitere besonders bevorzugte erfindungsgemäße Produkte sind gekennzeichnet durch einen Gehalt an mindestens einer Verbindung, die ein Inhibitor des Enzyms Cystathionin-beta-Lyase ist. Erfindungsgemäß bevorzugte Deodorantwirkstoffe, die als Inhibitor der Cystathionin-beta-Lyase wirken, sind ausgewählt aus denen, die in EP 495918 B1, WO 2006/079934, DE 102010000746 A1, WO2010/031657 A1 und WO2010/046291 A1 offenbart sind. Weitere besonders bevorzugte erfindungsgemäße Produkte sind gekennzeichnet durch einen Gehalt an mindestens einer Verbindung, die ein Inhibitor des Enzyms Cystathionin-beta-Lyase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Wasser-in-Öl-Emulsion, ohne das Gewicht des Treibmittels zu berücksichtigen.

Ein weiterer optionaler Bestandteil erfindungsgemäßer Produkte ist mindestens ein kationisches Phospholipid der Formel KPL, 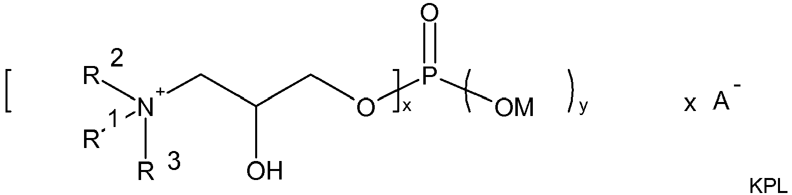 in der R¹ eine Alkyl-, Alkenyl- oder Hydroxyalkylgruppe mit 8 bis 22 C-Atomen oder eine Acylaminoalkylgruppe der Formel R⁵CONH(CₘH₂ₘ)- ist, worin R⁵CO eine lineare Acylgruppe mit 8 bis 22 C-Atomen und m = 2 oder 3 ist, R² und R³ Alkylgruppen mit 1 bis 4 C-Atomen oder Hydroxyalkylgruppen mit 2 bis 4 C- Atomen oder Carboxyalkylgruppen der Formel -(CH₂)_{z}-COOM sind, worin z einen Wert von 1 bis 3 hat und M Wasserstoff oder ein Alkalimetallkation ist, x einen Wert von 1 bis 3 und y einen Wert von (3 - x) hat, M Wasserstoff oder ein Alkalimetallkation ist und A⁻ein Anion ist.

Bevorzugte Alkylgruppen mit 8 bis 22 C-Atomen sind ausgewählt aus einer n-Octyl-, n-Nonyl-, n-Decyl-, n-Undecyl-, Lauryl-, n-Tridecanyl-, Myristyl-, n-Pentadecanyl-, Cetyl-, Palmityl-, Stearyl-, Elaidyl-, Arachidyl-, Behenyl- und einer Cocyl-Gruppe. Eine repräsentative Cocyl-Gruppe besteht, bezogen auf ihr Gesamtgewicht, aus 4 - 9 Gew.-% n-Octyl-, 4 - 9 Gew.-% n-Decyl-, 45 - 55 Gew.-% Lauryl-, 15 - 21 Gew.-% Myristyl-, 8 - 13 Gew.-% Palmityl- und 7 - 14 Gew.-% Stearyl-Gruppen. Bevorzugte Alkenylgruppen mit 8 bis 22 C-Atomen sind ausgewählt aus einer Linoleyl-Gruppe ((9*Z*,12*Z*)-Octadeca-9,12-dien-1-yl) und einer Linolenyl-Gruppe ((9Z,12Z,15Z)-Octadeca-9,12,15-trien-1-yl). Eine bevorzugte Hydroxyalkylgruppe mit 8 bis 22 C-Atomen ist ausgewählt aus einer 12-Hydroxystearylgruppe.

Besonders bevorzugte kationische Phospholipide der Formel KPL sind solche, bei denen R¹ eine Acylaminoalkylgruppe der Formel R⁵CONH(CₘH₂ₘ)- ist, worin R⁵CO eine lineare Acylgruppe mit 8 bis 22 C-Atomen darstellt und m = 3 ist.

Bevorzugte lineare Acylgruppen R⁵CO mit 8 bis 22 C-Atomen sind ausgewählt aus einer n-Octanoyl-, n-Nonanoyl-, n-Decanoyl-, n-Undecanoyl-, Lauroyl-, n-Tridecanoyl-, Myristoyl-, n-Pentadecanoyl-, Cetoyl-, Palmitoyl-, Stearoyl-, Elaidoyl-, Arachidoyl-, Behenoyl- und einer Cocoyl-Gruppe. Eine repräsentative Cocoyl-Gruppe besteht, bezogen auf ihr Gesamtgewicht, aus 4 - 9 Gew.-% n-Octanoyl-, 4 - 9 Gew.-% n-Decanoyl-, 45 - 55 Gew.-% Lauroyl-, 15 - 21 Gew.-% Myristoyl-, 8 - 13 Gew.-% Palmitoyl- und 7 - 14 Gew.-% Stearoyl-Gruppen. Besonders bevorzugte lineare Acylgruppen R⁵CO sind ausgewählt aus einer Cocoyl-Gruppe, einer Lauroyl-Gruppe (n-C₁₁H₂₃CO), einer Myristoyl-Gruppe (n-C₁₃H₂₇CO) und einer Linoleoyl-Gruppe ((9Z,12Z)-Octa-deca-9,12-dien-1-oyl). Außerordentlich bevorzugte lineare Acylgruppen R⁵CO sind ausgewählt aus einer Cocoyl-Gruppe, einer Lauroyl-Gruppe (n-C₁₁H₂₃CO) und einer Myristoyl-Gruppe (n-C₁₃H₂₇CO).

Bevorzugte Alkylgruppen mit 1 bis 4 C-Atomen sind eine Methyl-, Ethyl-, n-Propyl, 1-Methylethyl-, n-Butyl, 2-Methylpropyl- und eine tert.-Butyl-Gruppe. Besonders bevorzugt ist die Methylgruppe.

Bevorzugte Hydroxyalkylgruppen mit 2 - 4 C-Atomen sind eine 2-Hydroxyethyl- und eine 1-Hydroxyethylgruppe.

Bevorzugte Carboxyalkylgruppen der Formel -(CH₂)_{z}-COOM mit z = 1 bis 3 sind eine Carboxymethyl-, eine Carboxyethyl und eine Carboxy-n-propyl-Gruppe.

Bevorzugte Alkalimetallkationen sind ausgewählt aus Natrium- und Kalium-Kationen; Na⁺ ist besonders bevorzugt. Bevorzugte Anionen sind ausgewählt aus Sulfat, Chlorid, Phosphat, Nitrat, Hydrogencarbonat und Acetat, wobei ein Chloridanion besonders bevorzugt ist.

Bevorzugte erfindungsgemäße Produkte enthalten als deodorierenden Wirkstoff ein kationisches Phospholipid der Formel KPL, 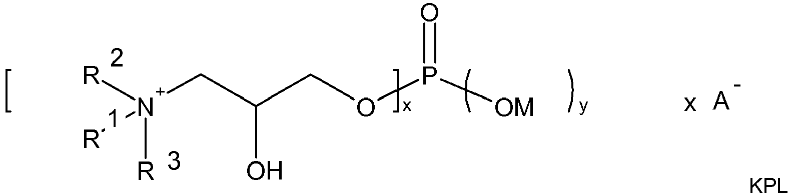 in der R¹ eine Acylaminoalkylgruppe der Formel R⁵CONH(CₘH₂ₘ)- ist, worin R⁵CO ausgewählt ist aus einer Cocoylgruppe, einer Lauroylgruppe, einer Myristoylgruppe und einer Linoleoyl-Gruppe und m = 3 ist, R² und R³ Methylgruppen sind, x = 2, y = 1, M ein Natriumion und A⁻ ein Chloridion sind. Bevorzugt ist mindestens ein kationisches Phospholipid der Formel KPL mit den vorstehend genannten Merkmalen in einer Gesamtmenge von 0,05 - 2 Gew.-%, bevorzugt 0,1 - 1 Gew.-%, besonders bevorzugt 0,15 - 0,4 Gew.-%, enthalten, jeweils bezogen auf das Gewicht der Wasser-in-Öl-Emulsion, ohne ggf. enthaltenes Treibmittel zu berücksichtigen.

Besonders bevorzugte erfindungsgemäße Produkte enthalten ein kationisches Phospholipid der Formel KPL, 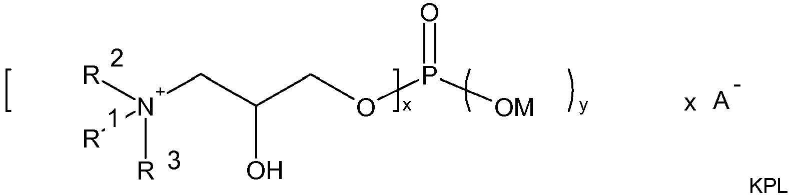 in der R¹ eine Cocoylaminopropylgruppe (auch als Cocamidopropylgruppe bezeichnet) ist, R² und R³ Methylgruppen sind, x = 2, y = 1, M ein Natriumion und A⁻ ein Chloridion sind und die unter der INCI-Bezeichnung Cocamidopropyl PG-Dimonium Chloride Phosphate erhältlich ist, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht der Wasser-in-Öl-Emulsion, ohne ggf. enthaltenes Treibmittel zu berücksichtigen.

Weitere besonders bevorzugte erfindungsgemäße Produkte enthalten ein kationisches Phospholipid der Formel KPL, 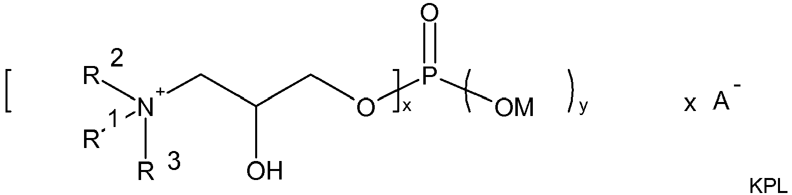 in der R¹ eine Myristoylaminopropylgruppe ist, R² und R³ Methylgruppen sind, x = 2, y = 1, M ein Natriumion und A⁻ ein Chloridion sind und die unter der INCI-Bezeichnung Myristamidopropyl PG-Dimonium Chloride Phosphate erhältlich ist, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht der Wasser-in-Öl-Emulsion, ohne ggf. enthaltenes Treibmittel zu berücksichtigen.

Weitere besonders bevorzugte erfindungsgemäße Produkte enthalten ein kationisches Phospholipid der Formel KPL, 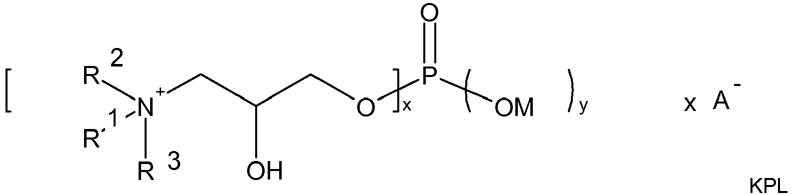 in der R¹ eine Lauroylaminopropylgruppe ist, R² und R³ Methylgruppen sind, x = 2, y = 1, M ein Natriumion und A⁻ ein Chloridion sind, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht der Wasser-in-Öl-Emulsion, ohne ggf. enthaltenes Treibmittel zu berücksichtigen.

Die erfindungsgemäße Wasser-in-Öl-Emulsion ist zusammen mit mindestens einem Treibmittel in einer Aerosol-Abgabevorrichtung enthalten; das erfindungsgemäße kosmetische Produkt besteht aus der Wasser-in-Öl-Emulsion wie vorstehend dargestellt, mindestens einem Treibmittel und einer Aerosol-Abgabevorrichtung.

Erfindungsgemäß geeignete Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase.

Weitere erfindungsgemäß geeignete und bevorzugte Treibmittel sind ausgewählt aus mindestens einer Verbindung mit 3 bis 10 Kohlenstoffatomen gemäß Formel (I) 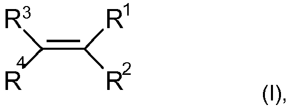 worin die Reste R¹, R², R³ und R⁴ unabhängig voneinander ein Wasserstoffatom, ein Bromatom, ein Fluoratom oder eine mit mindestens einem Fluoratom substituierte (C₁ bis C₆)-Alkylgruppe bedeuten, oder zwei der Reste R¹, R², R³ und R⁴ einen fünf- oder sechsgliedrigen Ring bilden, mit der Maßgabe, dass mindestens einer der Reste R¹, R², R³ oder R⁴ für ein Wasserstoffatom oder ein Fluoratom steht und mindestens einer der Reste R¹, R², R³ oder R⁴ für eine mit mindestens einem Fluoratom substituierte (C₁ bis C₆)-Alkylgruppe steht oder mindestens zwei der Reste R¹, R², R³ und R⁴ einen fünf- oder sechsgliedrigen Ring bilden.

Bevorzugte Treibmittel sind ausgewählt aus mindestens einer Verbindung mit 3 bis 10 Kohlenstoffatomen gemäß der vorstehenden Formel (I), in der R¹, R², R³ oder R⁴ unabhängig voneinander ein Wasserstoffatom, ein Fluoratom oder eine mit mindestens einem Fluoratom substituierte (C₁ bis C₆)-Alkylgruppe bedeuten, mit der Maßgabe, dass mindestens einer der Reste R¹, R², R³ oder R⁴ für ein Wasserstoffatom oder ein Fluoratom steht, und mindestens einer der Reste R¹, R², R³ oder R⁴ für eine mit mindestens einem Fluoratom substituierte (C₁ bis C₆)-Alkylgruppe steht.

Besonders bevorzugte Treibmittel sind ausgewählt aus Verbindungen der Formel *E*-R¹CH=CHR² oder der Formel Z-R¹CH=CHR², worin R¹ und R² unabhängig voneinander eine perfluorierte C₁- bis C₆-Alkylgruppe darstellen.

Weitere besonders bevorzugte Treibmittel sind ausgewählt aus CF₃CF=CHF, CF₃CH=CF₂, CHF₂CF=CF₂, CHF₂CH=CHF, CF₃CF=CH₂, CF₃CH=CHF, CH₂FCF=CF₂, CHF₂CH=CF₂, CHF₂CF=CHF, CHF₂CF=CH₂, CF₃CH=CH₂, CH₃CF=CF₂, CH₂FCH=CF₂, CH₂FCF=CHF, CHF₂CH=CHF, CF₃CF=CFCF₃, CF₃CF₂CF=CF₂, CF₃CF=CHCF₃, CF₃CF₂CF=CH₂, CF₃CH=CHF₃, CF₃CF₂CH=CH₂, CF₂=CHCF₂CF₃, CF₂=CHCF₂CF₃, CF₂=CFCHFCF₃, CF₂=CFCF₂CHF₂, CHF₂CH=CHCF₃, (CF₃)₂C=CHCF₃, CF₃CF=CHCF₂CF₃, CF₃CH=CFCF₂CF₃, (CF₃)₂CFCH=CH₂, CF₃CF₂CF₂CH=CH₂, CF₃(CF₂)₃CF=CF₂, CF₃CF₂CF=CFCF₂CF₃, (CF₃)₂C=C(CF₃)₂, (CF₃)₂CFCF=CHCF₃, CF₂=CFCF₂CH₂F, CF₂=CFCHFCHF₂, CH₂=C(CF₃)₂, CH₂CF₂CF=CF₂, CH₂FCF=CFCHF₂, CH₂FCF₂CF=CF₂, CF₂=C(CF₃)(CH₃), CH₂=C(CHF₂)(CF₃), CH₂=CHCF₂CHF₂, CF₂=C(CHF₂)(CH₃), CHF=C(CF₃)(CH₃), CH₂=C(CHF₂)₂, CF₃CF=CFCH₃, CH₃CF=CHCF₃, CF₂=CF(CF₂)₂CF₃, CHF=CF(CF₂)₂CF₃, CF₂=CH(CF₂)₂CF₃, CF₂=CF(CF₂)₂CHF₂, CHF₂CF=CFCF₂CF₃, CF₃CF=CFCF₂CHF₂, CF₃CF=CFCHFCF₃, CHF=CFCF(CF₃)₂, CF₂=CFCH(CF₃)₂, CF₃CH=C(CF₃)₂, CF₂=CHCF(CF₃)₂, CH2=CF(CF₂)₂CF₃, CHF=CF(CF₂)₂CHF₂, CH₂=C(CF₃)C₂F₅, CF₂=CHCH(CF₃)₂, CHF=CHCF(CF₃)₂, CF₂=C(CF₃)CH₂CF₃, CH₂=CF(CF₂)₂CHF₂, CF₂=CHCF₂CH₂CF₃, CF₃CF=C(CF₃)CH₃, CH₂=CFCH(CF₃)₂, CHF=CHCH(CF₃)₂, CH₂FCH=C(CF₃)₂, CH₃CF=C(CF₃)₂, CH₂=CHCF₂CHFCF₃, CH₂=C(CF₃)CH₂CF₃, (CF₃)₂C=CHC₂F₅, CH₂=CHC(CF₃)₃, (CF₃)₂C=C(CH₃)CF₃, CH₂=CFCF₂CH(CF₃)₂, CF₃CF=C(CH₃)C₂F₅, CF₃CH=CHCH(CF₃)₂, CH₂=CH(CF₂)₃CHF₂, (CF₃)₂C=CHCF₂CH₃, CH₂=C(CF₃)CH₂C₂F₅, CH₂=CHCH₂CF₂CF₂CF₃, C₂F₅CF=CFC₂H₅, CH₂=CHCH₂CF(CF₃)₂, CF₃CF=CHCH(CF₃)(CH₃), (CF₃)₂C=CFC₂H₅, cyclo-CF₂CF₂CF₂CH=CH-, cyclo-CF₂CF₂CH=CH-, CF₃CF₂CF₂C(CH₃)=CH₂, CF₃CF₂CF₂CH=CHCH₃, cyclo-CF₂CF₂CF=CF-, cyclo-CF₂CF=CFCF₂CF₂-, cyclo-CF₂CF=CFCF₂CF₂CF₂-, CF₃CF₂CF₂CF₂CH=CH₂, CF₃CH=CHC₂F₅, C₂F₅CH=CHC₂F₅, CF₃CH=CHCF₂CF₂CF₃, CF₃CF=CFC₂F₅, CF₃CF=CFCF₂CF₂CF₂CF₃, C₂F₅CF=CFCF₂CF₂CF₃, CF₃CH=CFCF₂CF₂CF₂CF₃, CF₃CF=CHCF₂CF₂CF₂CF₃, C₂F₅CH=CFCH₂CH₂CH₃, C₂F₅CF=CHCF₂CF₂CF₃, CF₃CF₂CF₂CF=CHCH₃, C₂F₅CF=CHCH₃, (CF₃)₂C=CHCH₃, CF₃C(CH₃)=CHCF₃, CHF=CFC₂F₅, CHF₂CF=CFCF₃, (CF₃)₂C=CHF, CH₂FCF=CFCF₃, CHF=CHC₂F₅, CHF₂CH=CFCF₃, CHF=CFCHFCF₃, CF₃CH=CFCHF₂, CHF=CFCF₂CHF₂, CHF₂CF=CFCHF₂, CH₂CF=CFCF₃, CH₂FCH=CFCF₃, CH₂=CFCHFCF₃, CH₂=CFCF₂CHF₂, CF₃CH=CFCH₂F, CHF=CFCH₂CF₃, CHF=CHCHFCF₃, CHF=CHCF₂CHF₂, CHF₂CF=CHCHF₂, CHF=CFCHFCHF₂, CF₃CF=CHCH₃, CF₂=CHCF₂Br, CHF=CBrCHF₂, CHBr=CHCF₃, CF₃CBr=CFCF₃, CH₂=CBrC₂F₅, CHBr=CHC₂F₅, CH₂=CH(CF₂)₂Br, CH₂=CHCBrFCF₃, CH₃CBr=CHCF₃, CF₃CBr=CHCH₃, (CF₃)₂C=CHBr, CF₃CF=CBrC₂F₅, *E*-CHF₂CBr=CFC₂F₅, *Z*-CHF₂CBr=CFC₂F₅, CF₂=CBrCHFC₂F₅, (CF₃)₂CFCBr=CH₂, CHBr=CF(CF₂)₂CHF₂, CH₂=CBrCF₂CF₂CF₃, CF₂=C(CH₂Br)CF₃, CH₂=C(CBrF₂)CF₃, (CF₃)₂CHCH=CHBr, (CF₃)₂C=CHCH₂Br, CH₂=CHCF(CF₃)CBrF₂, CF₂=CHCF₂CH₂CBrF₂, CFBr=CHCF₃, CFBr=CFCF₃ und CH₂=CBrCF₂CF₂CF₂CF₃, jeweils in der E-Form oder der Z-Form, sowie Mischungen der vorgenannten Komponenten.

Besonders bevorzugte erfindungsgemäße kosmetische Produkte enthalten als Treibmittel der Formel (I) das *E*-CF₃CH=CHF (*E*-1,3,3,3-Tetrafluorprop-1-en).

Kosmetische Produkte, die das Treibmittel in einer Menge von 10 - 90 Gew.-%, bevorzugt 40 - 85 Gew.-% und besonders bevorzugt 50 - 80 Gew.-%, jeweils bezogen auf das Gesamtgewicht der Zubereitung, bestehend aus der Wasser-in-Öl-Emulsion und dem Treibmittel, enthalten, sind erfindungsgemäß bevorzugt.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der Wasser-in-Öl-Emulsion.

Die erfindungsgemäßen Produkte weisen trotz der Wasserphase im Aerosolbehälter eine besonders hohe Korrosionsbeständigkeit auf, was einen großen Vorteil gegenüber dem Stand der Technik darstellt. Ferner weisen die erfindungsgemäß eingesetzten Wasser-in-Öl-Emulsionen eine ausgezeichnete Hautverträglichkeit, Lagerstabilität und Wirksamkeit auf. Vorteilhaft ist insbesondere, dass die versprühten Produkte sich auf der Haut durch ein angenehmes, nichtklebriges Hautgefühl mit einem lang anhaltenden Kühleffekt auszeichnen.

In einer bevorzugten Ausführungsform der Erfindung weist das Ventil einen mit einem Lack oder einem polymeren Kunststoff A beschichteten Ventilteller und ein ebensolches flexibles Element mit Rückstellcharakteristik auf, dass das Ventil nach Beenden der Betätigung in die Verschlussstellung (= Ruhelage des Ventils) zurückstellt. Entsprechende kosmetische Produkte, bei denen die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff A beschichtet ist/sind, sind erfindungsgemäß bevorzugt.

In einer weiteren bevorzugten Ausführungsform der Erfindung weist das Ventil ein flexibles Element mit Rückstellcharakteristik und/oder einen Ventilteller aus mindestens einem Kunststoff B, bevorzugt einem elastomeren Kunststoff, auf. Auch hier sind erfindungsgemäße kosmetische Produkte, bei denen das Ventil ein flexibles Element mit Rückstellcharakteristik und/oder einen Ventilkegel aus mindestens einem Kunststoff B aufweist, bevorzugt, wobei bevorzugte Kunststoffe B elastomere Kunststoffe sind. Besonders bevorzugte elastomere Kunststoffe sind ausgewählt aus Buna, insbesondere Buna N, Buna 421, Buna 1602 und Buna KA 6712, Neopren, Butyl und Chlorbutyl.

In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element mit Rückstellcharakteristik einstückig mit dem Ventilkegel ausgebildet sein und biegsame Beine aufweisen. Diese Feder kann aus Metall, bevorzugt aus hoch vergütetem, korrosionsstabilen Edelstahl (z. B Sorte AISI 316) oder Kunststoff sein.

In einer besonders bevorzugten Ausführungsform der Erfindung sind Ventilkegel und flexibles Element mit Rückstellcharakteristik ausgebildet. Besonders bevorzugt ist dabei der Ventiltyp Ariane M, erhältlich von der Firma Seaquist Perfect, bei dem das flexible Element mit Rückstellcharakteristik in Form von vier elastischen Beinen einstückig mit dem Ventilkegel ausgebildet ist. Alle erfindungsgemäß verwendeten Ventile weisen vorzugsweise einen innenlackierten Ventilteller auf, wobei Lackierung und Ventilmaterial miteinander kompatibel sind. Werden erfindungsgemäß Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Die eingesetzten Behälter, die z. B. aus Weißblech oder aus Aluminium sein können, wobei Aluminiumbehälter erfindungsgemäß bevorzugt sind, müssen angesichts der Korrosivität der erfindungsgemäß verwendeten Wasser-in-Öl-Emulsionen ebenfalls innen lackiert oder beschichtet sein. Ein erfindungsgemäß bevorzugter Innenschutzlack ist ein Epoxy-Phenollack, wie er u. a. unter den Bezeichnungen Hoba 7407 P und Hoba 7940 erhältlich ist.

Die erfindungsgemäßen kosmetischen Produkte eignen sich zur Behandlung der Haut. Ein weiterer Gegenstand der vorliegenden Anmeldung ist daher ein Verfahren zur kosmetischen Behandlung der Haut, dadurch gekennzeichnet, dass eine Wasser-in-Öl-Emulsion, enthaltend
a) mindestens ein schweißhemmendes Aluminiumsalz,
b) mindestens einen Wasser-in-Öl-Emulgator mit einem HLB-Wert im Bereich von 1,5 - 6,5,
c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
d) mindestens ein kosmetisches Öl, das kein Riechstoff und kein ätherisches Öl ist,
e) 5-Methyl-2-(1-methylethyl)cyclohexyl-N-ethyloxamat, bevorzugt (1R,2S,5R)-5-Methyl-2-(1-methylethyl)cyclohexyl-N-ethyloxamat,
mit Hilfe von mindestens einem Treibmittel aus einer Aerosol-Abgabevorrichtung auf die Haut aufgetragen wird. Bezüglich weiterer bevorzugter Ausführungsformen des erfindungsgemäßen Verfahrens gilt mutatis mutandis das zu den erfindungsgemäßen Produkten bzw. Wasser-in-Öl-Emulsionen Gesagte.

Die nachfolgenden Beispiele sollen den Gegenstand der Erfindung verdeutlichen, ohne ihn hierauf zu beschränken.

Erfindungsgemäße Wasser-in-Öl-Emulsionen (alle Mengenangaben in Gew.-%, bezogen auf das Gesamtgewicht der Wasser-in-Öl-Emulsion ohne Treibmittel)

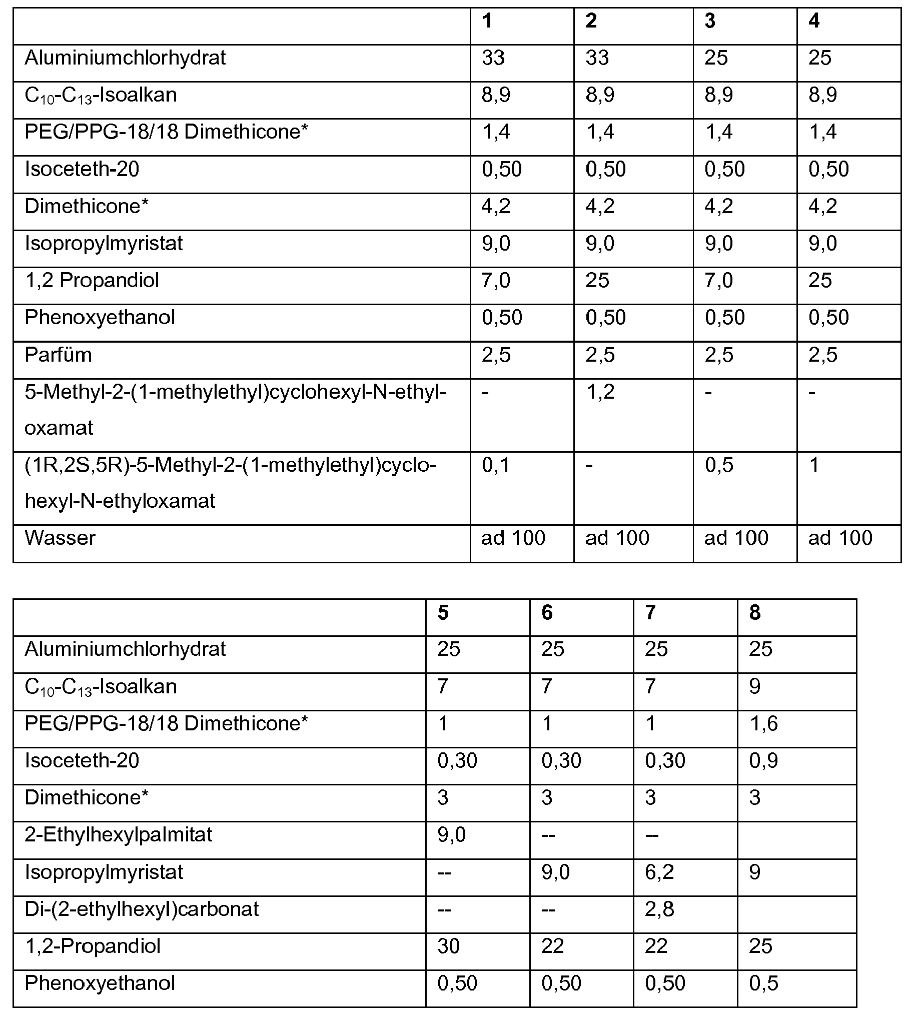

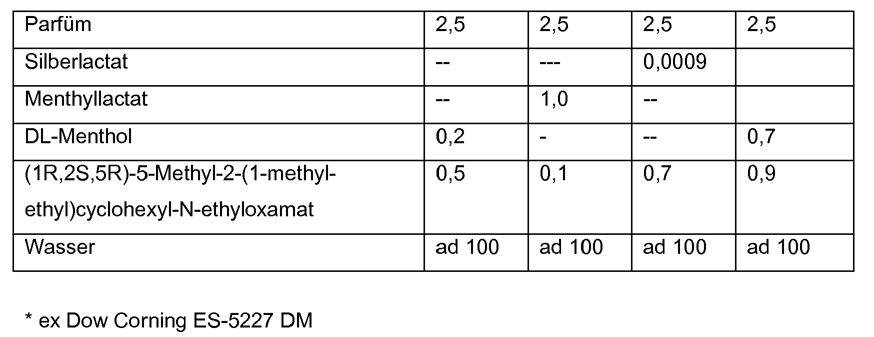

Die erfindungsgemäßen Wasser-in-Öl-Emulsionen Nr. 1 bis 8 werden in eine innen mit Epoxy-Phenollack beschichtete Aluminiumspraydose in einem Gewichtsverhältnis von Treibmittel (Butan/Propan/ Isobutangemisch) zu Emulsion von 80:20 bzw. 85:15 bzw. 60:40 zu einem erfindungsgemäßen Produkt abgefüllt. Nach dem Aufsprühen auf die Achselhaut hinterlassen sie einen lang anhaltenden Kühleffekt.

## Patentansprüche

1. Kosmetisches Produkt, bestehend aus
- einer Wasser-in-Öl-Emulsion, enthaltend
a) mindestens ein schweißhemmendes Aluminiumsalz,
b) mindestens einen Wasser-in-Öl-Emulgator mit einem HLB-Wert im Bereich von 1,5 bis 6,5,
c) mindestens einen Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17,
d) mindestens ein kosmetisches Öl, das kein Riechstoff und kein ätherisches Öl ist,
e) 5-Methyl-2-(1-methylethyl)cyclohexyl-N-ethyloxamat, bevorzugt (1R,2S,5R)-5-Ethyl-2-(1-methylethyl)cyclohexyl-N-ethyloxamat,
- mindestens einem Treibmittel,
- einer Aerosol-Abgabevorrichtung.

2. Produkt gemäß Anspruch 1, **dadurch gekennzeichnet, dass** 5-Methyl-2-(1-methylethyl)cyclohexyl-N-ethyloxamat, bevorzugt (1R,2S,5R)-5-Methyl-2-(1-methylethyl)cyclohexyl-N-ethyloxamat, in einer Gesamtmenge von 0,05 - 2 Gew.-%, bevorzugt 0,1 - 1,5 Gew.-%, besonders bevorzugt 0,2 - 1 Gew.-%, außerordentlich bevorzugt 0,3 - 0,5 Gew.-%, enthalten ist, wobei sich alle Mengenangaben auf das Gewicht der Wasser-in-Öl-Emulsion beziehen, ohne das Gewicht des Treibmittels zu berücksichtigen.

3. Produkt nach einem der vorherigen Ansprüche, **dadurch gekennzeichnet, dass** der Wasser-in-Öl-Emulgator b) ausgewählt ist aus Poly-(C₂-C₃)alkylenglycol-modifizierten Siliconen, bevorzugt ausgewählt aus Poly-(C₂-C₃)alkylenglycol-modifizierten Siliconen mit den INCI-Bezeichnungen PEG-x Dimethicone mit x = 2 - 20, bevorzugt 3 - 17, besonders bevorzugt 11 - 12, Bis-PEG-y Dimethicone mit y = 3 - 25, bevorzugt 4 - 20, PEG/PPG a/b Dimethicone, wobei a und b unabhängig voneinander für Zahlen von 2 - 30, bevorzugt 3 - 24, besonders bevorzugt 12 - 20, insbesondere 14 - 18, stehen, Bis-PEG/PPG-c/d Dimethicone, wobei c und d unabhängig voneinander für Zahlen von 10 - 25, bevorzugt 14 - 20 und besonders bevorzugt 14 - 16, stehen, und Bis-PEG/PPG-e/f PEG/PPG g/h Dimethicone, wobei e, f, g und h unabhängig voneinander für Zahlen von 10 - 20, bevorzugt 14 - 18 und besonders bevorzugt 16, stehen, mit der Bedingung, dass diese ethoxylierten und/oder propoxylierten Siliconemulgatoren unter Normalbedingungen einen HLB-Wert im Bereich von 1,5 bis 6,5, bevorzugt 4 bis 6,5, aufweisen, wobei PEG/PPG-18/18 Dimethicone mit einem HLB-Wert im Bereich von 4 bis 6,5 außerordentlich bevorzugt ist.

4. Produkt nach einem der vorherigen Ansprüche, **dadurch gekennzeichnet, dass** der mindestens eine Wasser-in-Öl-Emulgator b) in einer Gesamtmenge von 0,2 - 4,0 Gew.-%, bevorzugt 0,4 - 3,0 Gew.-% und insbesondere 0,7 - 2,0 Gew.-%, jeweils bezogen auf das Gewicht der gesamten treibmittelfreien Wasser-in-Öl-Emulsion, enthalten ist.

5. Produkt nach einem der vorherigen Ansprüche, **dadurch gekennzeichnet, dass** der mindestens eine Öl-in-Wasser-Emulgator oder Lösungsvermittler c) mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf das Gewicht der gesamten treibmittelfreien Wasser-in-Öl-Emulsion, enthalten ist.

6. Produkt nach einem der vorherigen Ansprüche, **dadurch gekennzeichnet, dass** der Öl-in-Wasser-Emulgator oder Lösungsvermittler mit einem HLB-Wert im Bereich von > 7 bis 20, bevorzugt im Bereich von > 12 bis 19, besonders bevorzugt im Bereich von > 14 bis 18 und außerordentlich bevorzugt im Bereich von 15 bis 17, ausgewählt ist aus nichtionischen Polyethylenglycolethern.

7. Produkt nach Anspruch 6, **dadurch gekennzeichnet, dass** der mindestens eine nichtionische Polyethylenglycolether ausgewählt ist aus der Gruppe der ethoxylierten C₈-C₂₄-Alkanole mit durchschnittlich 10 - 100 Mol Ethylenoxid pro Mol, bevorzugt aus der Gruppe der ethoxylierten C₁₂-C₁₈-Alkanole mit durchschnittlich 10 - 30 Mol Ethylenoxid pro Mol, besonders bevorzugt aus der Gruppe der ethoxylierten C₁₄-C₁₈-Alkanole mit durchschnittlich 20 - 30 Mol Ethylenoxid pro Mol.

8. Produkt nach einem der vorherigen Ansprüche, **dadurch gekennzeichnet, dass** der mindestens eine Öl-in-Wasser-Emulgator oder Lösungsvermittler mit einem HLB-Wert im Bereich von > 7 bis 20 ausgewählt ist aus Isoceteth-12, Isoceteth-20, Isoceteth-30, Isosteareth-12, Isosteareth-20, Isosteareth-30, Laureth-12 und Beheneth-20.

9. Produkt nach einem der vorherigen Ansprüche, **dadurch gekennzeichnet, dass** der mindestens eine Öl-in-Wasser-Emulgator oder Lösungsvermittler mit einem HLB-Wert im Bereich von > 7 bis 20 ausgewählt ist aus Isoceteth-20.

10. Produkt nach einem der vorherigen Ansprüche, **dadurch gekennzeichnet, dass** Isoceteth-20 in einer Gesamtmenge von 0,1 - 2,0 Gew.-%, bevorzugt 0,2 - 1,2 Gew.-% und besonders bevorzugt 0,5 - 0,8 Gew.-%, jeweils bezogen auf die gesamte treibmittelfreie Wasser-in-Öl-Emulsion, enthalten ist.

11. Produkt nach einem der vorherigen Ansprüche, **dadurch gekennzeichnet, dass** das mindestens eine kosmetische Öl d) in einer Gesamtmenge von 1 - 30 Gew.-%, bevorzugt 5 - 26 Gew.-%, besonders bevorzugt 9 - 24 Gew.-%, außerordentlich bevorzugt 15 - 20 Gew.-%, jeweils bezogen auf das Gewicht der gesamten treibmittelfreien Wasser-in-Öl-Emulsion, enthalten ist.

12. Produkt gemäß einem der vorherigen Ansprüche, **dadurch gekennzeichnet, dass** das kosmetische Öl d) mindestens ein flüchtiges Öl mit einem Dampfdruck von 10 - 3000 Pa bei 20°C umfasst, bevorzugt in einer Gesamtmenge von 10 - 100 Gew.-%, besonders bevorzugt 30 - 80 Gew.-%, jeweils bezogen auf das Gesamtgewicht der kosmetischen Öle d).

13. Produkt nach einem der vorherigen Ansprüche, **dadurch gekennzeichnet, dass** das flüchtige Öl d) mit einem Dampfdruck von 10 - 3000 Pa bei 20°C ausgewählt ist aus C₈-C₁₆-Isoparaffinen, bevorzugt ausgewählt aus Isooctan, Isononan, Isodecan, Isoundecan, Isododecan, Isotridecan, Isotetradecan, Isopentadecan, und Isohexadecan, sowie Mischungen hiervon, wobei C₁₀-C₁₃-Isoparaffin-Mischungen, insbesondere solche mit einem Dampfdruck bei 20°C von 10 bis 400 Pa besonders bevorzugt sind.

14. Produkt gemäß einem der vorherigen Ansprüche, **dadurch gekennzeichnet, dass** mindestens ein Kühlwirkstoff, ausgewählt aus L-Menthol, D-Menthol und DL-Menthol, bevorzugt L-Menthol, weiterhin aus Isopulegol, insbesondere (-)-Isopulegol (FEMA 2962), N-2,3-Trimethyl-2-Isopropylbutamid, Menthyllactat, Menthylpyrrolidoncarbonsäure, Menthylmethylether, Menthoxypropan-1,2-diol, Menthonglycerinacetal (9-Methyl-6-(1-methylethyl)-1,4-dioxaspiro(4.5) - decan-2-methanol), Monomenthylsuccinat, 2-Hydroxymethyl-3,5,5-trimethylcyclohexanol, 2-Isopropyl-N,2,3-trimethylbutyramid (FEMA 3804), N-Ethyl-p-menthan-3-carboxamid (FEMA 3455), insbesondere 1R,3R,4S-N-Ethyl-p-menthane-3-carboxamid, Ethyl-3-(p-menthan-3-carboxamido)acetat (FEMA 4309), (1R,2S,5R)-N-(4-Methoxyphenyl)-p-menthancarboxamid (FEMA 4681), N-Ethyl-2,2-diisopropylbutanamid (FEMA 4557), N-Cyclopropyl-5-methyl-2-propan-2-ylcyclohexan-1-carboxamid (FEMA 4693), N-(4-Cyanomethylphenyl)-p-menthan-carboxamid (FEMA 4496), N-(2-(Pyridin-2-yl)ethyl)-3-p-menthancarboxamid (FEMA 4549), N-(2-Hydroxyethyl)-2-isopropyl-2,3-dimethylbutanamid (FEMA 4602), N-(1,1-Dimethyl-2-hydroxyethyl)-2,2-diethylbutanamid (FEMA 4603), (2S,5R)-N-[4-(2-Amino-2-oxoethyl)phenyl]-p-menthancarboxamid (FEMA 4684), 2-[(2-p-Menthoxy)ethoxy]ethanol (FEMA 4718), (2,6-Diethyl-5-isopropyl-2-methyltetrahydropyran (FEMA 4680), 3-(I-Menthoxy)-2-methylpropan-1,2-diol (FEMA 3849), p-Menthan-3,8-diol, insbesondere (+)-cis-p-Menthan-3,8-diol und (-)-trans-p-Menthan-3,8-diol sowie Mischungen aus (+)-cis-p-Menthan-3,8-diol und (-)-trans-p-Menthan-3,8-diol, insbesondere eine Mischung im Gewichtsverhältnis 62:38 (FEMA 4053), (1 R,3R,4S)-3-Menthyl-3,6-dioxaheptanoat, (1 R,2S,5R)-3-Menthylmethoxyacetat, (1 R,2S,5R)-3-Menthyl-3,6,9-trioxadecanoat, (1 R,2S,5R)-3-Menthyl-3,6,9-trioxadecanoat, (1R,2S,5R)-3-Menthyl-(2-hydroxyethoxy)acetat, (1R,2S,5R)-Menthyl-11-hydroxy-3,6,9-trioxa-undecanoat, (-)-Cubebol (FEMA 4497), N-(4-Cyanomethylphenyl)-p-menthancarboxamid, N,N-Dimethylmenthylsuccinamid (2-Isopropyl-5-methylcyclohexyl-4-(dimethylamino)-4-oxo-butanoat, FEMA 4230), 6-Isopropyl-3,9-dimethyl-1,4-dioxaspiro[4.5]decan-2-on (FEMA 4285), sowie Mischungen dieser Substanzen, enthalten ist, wobei L-Menthol besonders bevorzugt ist.

15. Produkt gemäß einem der vorherigen Ansprüche, **dadurch gekennzeichnet, dass** das mindestens eine schweißhemmende Aluminiumsalz in einer Gesamtmenge von 5 bis 40 Gew.-%, bevorzugt 10 bis 30 Gew.-%, besonders bevorzugt 15 bis 25 Gew.-% und außerordentlich bevorzugt 20 bis 23 Gew.-%, jeweils bezogen auf das Gesamtgewicht der kristallwasser- und ligandenfreien Aktivsubstanz (USP) in der treibmittelfreien Wasser-in-Öl-Emulsion, enthalten ist.

## Claims

1. A cosmetic product consisting of
- a water-in-oil emulsion containing
a) at least one sweat-inhibiting aluminum salt,
b) at least one water-in-oil emulsifier having an HLB value in the range from 1.5 to 6.5,
c) at least one oil-in-water emulsifier or solubilizer having an HLB value in the range from > 7 to 20, preferably in the range from > 12 to 19, particularly preferably in the range from > 14 to 18 and exceptionally preferably in the range from 15 to 17,
d) at least one cosmetic oil that is not a fragrance and not an essential oil,
e) 5-methyl-2-(1-methylethyl)cyclohexyl-N-ethyl oxamate, preferably (1 R,2S,5R)-5-methyl-2-(1-methylethyl)cyclohexyl-N-ethyl oxamate,
- at least one propellant,
- an aerosol dispensing device.

2. The product according to claim 1, **characterized in that** 5-methyl-2-(1-methylethyl)cyclohexyl-N-ethyl oxamate, preferably (1 R,2S,5R)-5-methyl-2-(1-methylethyl)cyclohexyl-N-ethyl oxamate, is included in a total amount from 0.05 to 2 wt.%, preferably 0.1 to 1.5 wt.%, particularly preferably 0.2 to 1 wt.%, exceptionally preferably 0.3 to 0.5 wt.%, wherein all stated amounts relate to the weight of the water-in-oil emulsion, without taking into account the weight of the propellant.

3. The product according to one of the preceding claims, **characterized in that** the water-in-oil emulsifier b) is selected from poly-(C₂-C₃)alkylene glycol-modified silicones, preferably selected from poly-(C₂-C₃)alkylene glycol-modified silicones having the INCI names PEG-x dimethicone with x = 2 - 20, preferably 3 - 17, particularly preferably 11 - 12, bis-PEG-y dimethicone with y = 3 - 25, preferably 4 - 20, PEG/PPG-a/b dimethicone, where a and b independently of one another denote numbers from 2 - 30, preferably 3 - 24, particularly preferably 12 - 20, in particular 14 - 18, bis-PEG/PPG-c/d dimethicone, where c and d independently of one another denote numbers from 10 - 25, preferably 14 - 20 and particularly preferably 14 - 16, and bis-PEG/PPG-e/f PEG/PPG-g/h dimethicone, where e, f, g and h independently of one another denote numbers from 10 - 20, preferably 14 - 18 and particularly preferably 16, on condition that these ethoxylated and/or propoxylated silicone emulsifiers have an HLB value under normal conditions in the range from 1.5 to 6.5, preferably 4 to 6.5, wherein PEG/PPG-18/18 dimethicone with an HLB value in the range from 4 to 6.5 is exceptionally preferred.

4. The product according to one of the preceding claims, **characterized in that** the at least one water-in-oil emulsifier b) is included in a total amount from 0.2 to 4.0 wt.%, preferably 0.4 to 3.0 wt.% and in particular 0.7 to 2.0 wt.%, relative in each case to the weight of the total propellant-free water-in-oil emulsion.

5. The product according to one of the preceding claims, **characterized in that** the at least one oil-in-water emulsifier or solubilizer c) having an HLB value in the range from > 7 to 20, preferably in the range from > 12 to 19, particularly preferably in the range from > 14 to 18 and exceptionally preferably in the range from 15 to 17 is included in a total amount from 0.1 to 2.0 wt.%, preferably 0.2 to 1.2 wt.% and particularly preferably 0.5 to 0.8 wt.%, relative in each case to the weight of the total propellant-free water-in-oil emulsion.

6. The product according to one of the preceding claims, **characterized in that** the oil-in-water emulsifier or solubilizer having an HLB value in the range from > 7 to 20, preferably in the range from > 12 to 19, particularly preferably in the range from > 14 to 18 and exceptionally preferably in the range from 15 to 17 is selected from non-ionic polyethylene glycol ethers.

7. The product according to claim 6, **characterized in that** the at least one non-ionic polyethylene glycol ether is selected from the group of ethoxylated C₈-C₂₄ alkanols having on average 10 to 100 mol of ethylene oxide per mol, preferably from the group of ethoxylated C₁₂-C₁₈ alkanols having on average 10 to 30 mol of ethylene oxide per mol, particularly preferably from the group of ethoxylated C₁₄-C₁₈ alkanols having on average 20 to 30 mol of ethylene oxide per mol.

8. The product according to one of the preceding claims, **characterized in that** the at least one oil-in-water emulsifier or solubilizer having an HLB value in the range from > 7 to 20 is selected from Isoceteth-12, Isoceteth-20, Isoceteth-30, Isosteareth-12, Isosteareth-20, Isosteareth-30, Laureth-12 and Beheneth-20.

9. The product according to one of the preceding claims, **characterized in that** the at least one oil-in-water emulsifier or solubilizer having an HLB value in the range from > 7 to 20 is selected from Isoceteth-20.

10. The product according to one of the preceding claims, **characterized in that** Isoceteth-20 is included in a total amount from 0.1 to 2.0 wt.%, preferably 0.2 to 1.2 wt.% and particularly preferably 0.5 to 0.8 wt.%, relative in each case to the total propellant-free water-in-oil emulsion.

11. The product according to one of the preceding claims, **characterized in that** the at least one cosmetic oil d) is included in a total amount from 1 to 30 wt.%, preferably 5 to 26 wt.%, particularly preferably 9 to 24 wt.%, exceptionally preferably 15 to 20 wt.%, relative in each case to the weight of the total propellant-free water-in-oil emulsion.

12. The product according to one of the preceding claims, **characterized in that** the cosmetic oil d) comprises at least one volatile oil having a vapor pressure from 10 to 3000 Pa at 20°C, preferably in a total amount from 10 to 100 wt.%, particularly preferably 30 to 80 wt.%, relative in each case to the total weight of the cosmetic oils d).

13. The product according to one of the preceding claims, **characterized in that** the volatile oil d) having a vapor pressure from 10 to 3000 Pa at 20°C is selected from C₈-C₁₆ isoparaffins, preferably selected from isooctane, isononane, isodecane, isoundecane, isododecane, isotridecane, isotetradecane, isopentadecane and isohexadecane, and mixtures thereof, wherein C₁₀-C₁₃ isoparaffin mixtures, in particular those having a vapor pressure at 20°C from 10 to 400 Pa, are particularly preferred.

14. The product according to one of the preceding claims, **characterized in that** at least one cooling active ingredient is included, selected from L-menthol, D-menthol and DL-menthol, preferably L-menthol, moreover from isopulegol, in particular (-)-isopulegol (FEMA 2962), N-2,3-trimethyl-2-isopropylbutamide, menthyl lactate, menthyl pyrrolidone carboxylic acid, menthyl methyl ether, menthoxypropane-1,2-diol, menthone glycerol acetal (9-methyl-6-(1-methylethyl)-1,4-dioxaspiro(4.5)-decane-2-methanol), monomenthyl succinate, 2-hydroxymethyl-3,5,5-trimethylcyclohexanol, 2-isopropyl-N,2,3-trimethylbutyramide (FEMA 3804), N-ethyl-p-menthane-3-carboxamide (FEMA 3455), in particular 1 R,3R,4S-N-ethyl-p-menthane-3-carboxamide, ethyl-3-(p-menthane-3-carboxamido)acetate (FEMA 4309), (1R,2S,5R)-N-(4-methoxyphenyl)-p-menthane carboxamide (FEMA 4681), N-ethyl-2,2-diisopropylbutanamide (FEMA 4557), N-cyclopropyl-5-methyl-2-propan-2-ylcyclohexane-1-carboxamide (FEMA 4693), N-(4-cyanomethylphenyl)-p-menthane carboxamide (FEMA 4496), N-(2-(pyridin-2-yl)ethyl)-3-p-menthane carboxamide (FEMA 4549), N-(2-hydroxyethyl)-2-isopropyl-2,3-dimethylbutanamide (FEMA 4602), N-(1,1-dimethyl-2-hydroxyethyl)-2,2-diethylbutanamide (FEMA 4603), (2S,5R)-N-[4-(2-amino-2-oxoethyl)phenyl]-p-menthane carboxamide (FEMA 4684), 2-[(2-p-menthoxy)ethoxy]ethanol (FEMA 4718), (2,6-diethyl-5-isopropyl-2-methyltetrahydropyrane (FEMA 4680), 3-(I-menthoxy)-2-methylpropane-1,2-diol (FEMA 3849), p-menthane-3,8-diol, in particular (+)-cis-p-menthane-3,8-diol and (-)-trans-p-menthane-3,8-diol as well as mixtures of (+)-cis-p-menthane-3,8-diol and (-)-trans-p-menthane-3,8-diol, in particular a mixture in the weight ratio 62:38 (FEMA 4053), (1R,3R,4S)-3-menthyl-3,6-dioxaheptanoate, (1R,2S,5R)-3-menthylmethoxyacetate, (1R,2S,5R)-3-menthyl-3,6,9-trioxadecanoate, (1 R,2S,5R)-3-menthyl-3,6,9-trioxadecanoate, (1 R,2S,5R)-3-menthyl-(2-hydroxyethoxy)acetate, (1 R,2S,5R)-menthyl-11-hydroxy-3,6,9-trioxaundecanoate, (-)-cubebol (FEMA 4497), N-(4-cyanomethylphenyl)-p-menthane carboxamide, N,N-dimethylmenthylsuccinamide (2-isopropyl-5-methylcyclohexyl-4-(dimethylamino)-4-oxobutanoate, FEMA 4230), 6-isopropyl-3,9-dimethyl-1,4-dioxaspiro[4.5]decan-2-one (FEMA 4285), as well as mixtures of these substances, L-menthol being particularly preferred.

15. The product according to one of the preceding claims, **characterized in that** the at least one sweat-inhibiting aluminum salt is included in a total amount from 5 to 40 wt.%, preferably 10 to 30 wt.%, particularly preferably 15 to 25 wt.% and exceptionally preferably 20 to 23 wt.%, relative in each case to the total weight of the active substance (USP), free from water of crystallization and ligands, in the propellant-free water-in-oil emulsion.

## Revendications

1. Produit cosmétique constitué par
- une émulsion eau-dans-huile, contenant
a) au moins un sel d'aluminium anti-transpirant,
b) au moins un émulsifiant eau-dans-huile ayant une valeur HLB dans la gamme de 1,5 à 6,5,
c) au moins un émulsifiant huile-dans-eau ou solubilisant ayant une valeur HLB dans la gamme allant de >7 à 20, de préférence dans la gamme allant de >12 à 19, de manière particulièrement préférée dans la gamme allant de >14 à 18 et de manière extraordinairement préférée dans la gamme allant de 15 à 17,
d) au moins une huile cosmétique qui n'est ni une substance odorante ni un huile essentielle,
e) du 5-méthyl-2-(1-méthyléthyl)cyclohexyl-N-éthyloxamate, de préférence du (1R,2S,5R)-5-méthyl-2-(1-méthyléthyl)cyclohexyl-N-éthyloxamate,
- au moins un agent propulseur,
- un dispositif de distribution d'aérosol.

2. Produit selon la revendication 1, **caractérisé en ce que** le 5-méthyl-2-(1-méthyléthyl)cyclohexyl-N-éthyloxamate, de préférence le (1R,2S,5R)-5-méthyl-2-(1-méthyléthyl)cyclohexyl-N-éthyloxamate est contenu, dans une quantité totale de 0,05 à 2% en poids, de préférence de 0,1 à 1,5% en poids, de manière particulièrement préférée de 0,2 à 1% en poids, de manière extraordinairement préférée de 0,3 à 0,5% en poids, toutes les données quantitatives se rapportant sur le poids de l'émulsion eau-dans-huile, sans tenir compte du poids de l'agent propulseur.

3. Produit selon l'une des revendications précédentes, **caractérisé en ce que** l'émulsifiant eau-dans-huile est choisi parmi les silicones modifiées par du polyalkylèneglycol en C₂ à C₃, de préférence choisie parmi les silicones modifiées par polyalkylèneglycol en C₂ à C₃ ayant les noms INCl PEG-x Dimethicone avec x = 2 à 20, de préférence 3 à 17, de manière particulièrement préférée de 11 à 12, Bis-PEG-y Dimethicone avec y = 3 à 25, de préférence 4 à 20, PEG/PPG a/b Dimethicone, a et b étant indépendamment un nombre de 2 à 30, de préférence de 3 à 24, de manière particulièrement préférée de 12 à 20, en particulier de 14 à 18, Bis-PEG/PPG-c/d Dimethicone, c et d étant indépendamment des nombres de 10 à 25, de préférence de 14 à 20 et de manière particulière préférée de 14 à 16, et Bis-PEG/PPG e/f et PEG/PPG g/g Dimethicone, e, f, g et h étant indépendamment des nombres de 10 à 20, de préférence de 14 à 18 et de manière particulièrement préférée le nombre 16, à la condition que ces émulsifiants siliconés éthoxylés et/ou propoxylés aient dans des conditions normales une valeur HLB dans la gamme allant de 1,5 à 6,5, de préférence de 4 à 6,5, PEG/PPG-18/18 Dimethicone ayant une valeur HLB dans la gamme de 4 à 6,5 étant extraordinairement préféré.

4. Produit selon l'une des revendications précédentes, **caractérisé en ce que** l'au moins un émulsifiant eau-dans-huile b) est contenu dans une quantité totale de 0,2 à 4,0% en poids, de préférence de 0,4 à 3,0% en poids et en particulier de 0,7 à 2,0% en poids, à chaque fois par rapport au poids de toute l'émulsion eau-dans-huile sans agent propulseur.

5. Produit selon l'une des revendications précédentes, **caractérisé en ce que** l'au moins un émulsifiant huile-dans-eau ou solubilisant c) ayant une valeur HLB dans la gamme de >7 à 20, de préférence dans la gamme de >12 à 19, de manière particulièrement préférée dans la gamme de >14 à 18 et de manière extraordinairement préférée dans la gamme de 15 à 17, est contenu dans une quantité totale de 0,1 à 2,0% en poids, de préférence de 0,2 à 1,2% en poids et de manière particulièrement préférée de 0,5 à 0,8% en poids, à chaque fois par rapport au poids de toute l'émulsion eau-dans-huile sans agent propulseur.

6. Produit selon l'une des revendications précédentes, **caractérisé en ce que** l'émulsifiant huile-dans-eau ou le solubilisant ayant une valeur HLB dans la gamme de > 7 à 20, de préférence dans la gamme de >12 à 19, de façon particulièrement préférée dans la gamme de >14 à 18 et de manière extraordinairement préférée dans la gamme de 15 à 17, est choisi parmi les éthers de polyéthylène-glycol non ioniques.

7. Produit selon la revendication 6, **caractérisé en ce que** l'au moins un éther de polyéthylène-glycol non-ionique est choisi dans le groupe des alcanols en C₈ à C₂₄ éthoxylés ayant en moyenne 10 à 100 moles d'oxyde d'éthylène par mole, de préférence dans le groupe des alcanols en C₁₂ à C₁₈ éthoxylés ayant en moyenne 10 à 30 moles d'oxyde d'éthylène par mole, de façon particulièrement préférée dans le groupe des alcanols en C₁₄ à C₁₈ éthoxylés ayant en moyenne 20 à 30 moles d'oxyde d'éthylène par mole.

8. Produit selon l'une des revendications précédentes, **caractérisé en ce que** l'au moins un émulsifiant huile-dans-eau ou solubilisant ayant une valeur HLB dans la gamme de >7 à 20 est choisi parmi l'isoceteth-12, l'isoceteth-20, l'isoceteth-30, l'isostéareth-12, l'isostéareth-20, l'isostéareth-30, le laureth-12 et le beheneth-20.

9. Produit selon l'une des revendications précédentes, **caractérisé en ce que** l'au moins un émulsifiant huile-dans-eau ou solubilisant ayant une valeur HLB dans la gamme de >7 à 20 est l'isoceteth-20.

10. Produit selon l'une des revendications précédentes, **caractérisé en ce que** l'isoceteth-20 est contenu dans une quantité totale de 0,1 à 2,0% en poids, de préférence de 0,2 à 1,2% en poids et de manière particulièrement préférée de 0,5 à 0,8% en poids, à chaque fois par rapport à toute l'émulsion eau-dans-huile sans gaz propulseur.

11. Produit selon l'une des revendications précédentes, **caractérisé en ce que** l'au moins une huile cosmétique d) est contenue dans une quantité totale de 1 à 30% en poids, de préférence de 5 à 26% en poids , de manière particulièrement préférée de 9 à 24% en poids, de manière extraordinairement préférée de 15 à 20% en poids, à chaque fois par rapport à toute l'émulsion eau-dans-huile sans gaz propulseur.

12. Produit selon l'une des revendications précédentes, **caractérisé en ce que** l'huile cosmétique d) comporte au moins une huile volatile ayant une pression de vapeur de 10 à 3000 Pa à 20°C, de préférence dans une quantité totale de 10 à 100% en poids, de manière particulièrement préférée de 30 à 80% en poids, à chaque fois par rapport au poids total de l'huile cosmétique d).

13. Produit selon l'une des revendications précédentes, **caractérisé en ce que** l'huile volatil d) ayant une pression de vapeur de 10 à 3000 Pa à 20°C est choisie parmi les isoparaffines en C₈ à C₁₆, de préférence l'isooctane, l'isononane, l'isodécane, l'isoundécane, l'isododécane, l'isotridécane, l'isotétradécane, l'isopentadécane, l'isohexadécane, et leurs mélanges, des mélanges d'isoparaffines en C₁₀ à C₁₃, en particulier ceux ayant une pression de vapeur à 20°C de 10 à 400 Pa, étant particulièrement préférés.

14. Produit selon l'une des revendications précédentes, **caractérisé en ce qu'**au moins une substance réfrigérante est choisie parmi le L-menthol, le D-menthol et le DL-menthol, de préférence le L-menthol, en outre parmi l'isopulégol, en particulier le (-)-isopulegol (FEMA 2962), le N-2,3-triméthyl-2-isopropylbutamide, le lactate de menthyle, l'acide menthylpyrrolidoncarboxylique, l'éther de menthylméthyle, le menthoxypropane-1,2-diol, l'acétal de menthonglycérol (9-méthyl-6-(1-méthyléthyl)-1,4-dioxaspiro(4,5)-décan-2-méthanol), le succinate de mono-menthyle, le 2-hydroxyméthyl-3,5,5-triméthylcyclohexanol, le 2-isopropyl-N,2,3-triméthylbutyramide (FEMA 3804), le N- éthyl-p-menthane-3-carboxamide (FEMA 3455), et notamment le 1R,3R,4S-N-éthyl-p-menthane-3-carboxamide, l'éthyl-3-(p-menthane-3-carboxamido)acétate (FEMA 4309), le (1R,2S,5R)-N- (4-méthoxyphényl)-p-menthane-carboxamide (FEMA 4681), le N-éthyl-2,2-diisopropylbutanamide (FEMA 4557), le N-cyclopropyl-5-méthyl-2-propan-2-ylcyclohexan-1-carboxamide (FEMA 4693), le N-(4-cyanométhylphényl)-p-menthane-carboxamide (FEMA 4496), le N-(2-(pyridin-2-yl)éthyl)-3-p-menthane-carboxamide (FEMA 4549), le N-(2-hydroxyéthyl)-2-isopropyl-2,3-diméthylbutanamide (FEMA 4602), le N-(1,1-diméthyl-2-hydroxyéthyl)-2,2-diéthylbutanamide (FEMA 4603), le (2S,5R)-N-[4-(2-amino-2-oxoéthyl)phényl]-p-menthane-carboxamide (FEMA 4684), le 2-[(2-p-menthoxy)éthoxy]éthanol (FEMA 4718), le (2,6-diéthyl-5-isopropyl-2-méthyltétrahydropyranne (FEMA 4680), le 3-(1-menthoxy)-2-méthylpropan-1,2-diol (FEMA 3849), le p-menthane3,8-diol, en particulier le (+)-cis-p-menthan-3,8-diol et le (-)-trans-p-menthan-3,8-diol et des mélanges de (+)-cis-p-menthan-3,8-diol et de (-)-trans-p-menthan-3,8-diol, en particulier un mélange dans un rapport en poids de 62:38 (FEMA 4053), le (1R,3R,4S)-3-menthyl-3,6-dioxaheptanoate, le (1R,2S,5R)-3-menthylméthoxyacétate, le (1 R,2S,5R)-3-menthyl-3,6,9-trioxadécanoate, le (1 R,2S,5R)-3-menthyl-3,6,9-trioxadécanoate, le (1R,2S,5R)-3-menthyl-(2-hydroxyéthoxy)acétate, le (1R,2S,5R)-menthyl-11-hydroxy-3,6,9-trioxa-undécanoate, le (-)-cubébol (FEMA 4497), le N- (4-cyanométhylphényl)-p-menthanecarboxamide, le N,N-diméthylmenthylsuccinamide (2-isopropyl-5-méthylcyclohexyl-4- (diméthylamino)-4-oxo-butanoate, FEMA 4230), le 6-isopropyl-3,9-diméthyl-1,4-dioxaspiro[4.5]décan-2-one (FEMA 4285), et des mélanges de ces substances, le L-menthol étant particulièrement préféré.

15. Produit selon l'une des revendications précédentes, **caractérisé en ce que** l'au moins un sel d'aluminium anti-transpirant est contenu dans une quantité totale de 5 à 40% en poids, de préférence de 10 à 30% en poids, de manière particulièrement préférée de 15 à 25% en poids et de manière extraordinairement préférée de 20 à 23% en poids, à chaque fois par rapport au poids total de la substance active sans ligand ni eau cristalline (USP) dans l'émulsion eau-dans-huile sans agent propulseur.
